# EUROPEAN PATENT APPLICATION

(11) **EP 4 744 662 A1**
(43) Date of publication of application: **20.05.2026**
(21) Application number: 24932088.8
(22) Date of filing: 02.10.2024
(51) Int. Cl.: A61K 31/495, A61K 31/138, A61K 31/215, A61K 31/675, A61K 45/06, A61P 31/14

(54) **ANTIVIRAL PHARMACEUTICAL COMPOSITION COMPRISING ERRy INHIBITOR AS ACTIVE INGREDIENT**

(30) Priority: 27.03.2024 KR 20240041829
(71) Applicant: Industry Foundation of Chonnam National University, Gwangju 61186 (KR); Novmetapharma Co., Ltd., Seoul 06050 (KR)
(72) Inventor: CHO, Kyoung-Oh, Gwangju 61185 (KR); CHOI, Hueng-Sik, Gwangju 61176 (KR); BAEK, Yeong-Bin, Seoul 01192 (KR); KWON, Hyung-Jun, Sejong 30147 (KR); KIM, Don-Kyu, Gwangju 61212 (KR); LEE, Sunwoo, Gwangju 61026 (KR); JUNG, Hoe-Yune, Pohang-si, Gyeongsangbuk-do 37668 (KR)
(74) Representative: Manitz Finsterwald Patent- und Rechtsanwaltspartnerschaft mbB
(86) International application number: PCT/KR2024/014934
(87) International publication number: WO 2025/206489

(57) **Abstract**

The present disclosure relates to an antiviral pharmaceutical composition including an estrogen-related receptor γ (ERRy) inhibitor as an active ingredient. According to embodiments of the present disclosure, an antiviral pharmaceutical composition containing an ERRy inhibitor as an active ingredient has antiviral effects. In particular, the antiviral pharmaceutical composition is effective in preventing or treating RNA virus infections. Specifically, the ERRy inhibitor, especially an ERRγ inverse agonist, may exhibit broad-spectrum antiviral effects by inhibiting ERRy activity in RNA virus-infected cells, thereby blocking fatty acid biosynthesis mediated by sterol regulatory element-binding protein 1c (SREBP1c).

## Description

### TECHNICAL FIELD

The present disclosure relates to an antiviral pharmaceutical composition including an estrogen-related receptor γ (ERRy) inhibitor as an active ingredient.

### BACKGROUND

The COVID-19 pandemic has emphasized the importance of zoonotic viral pathogens like influenza A virus (IAV), Ebola virus, and Zika virus. These RNA viruses have RNA-dependent RNA polymerases that lack exonuclease proofreading activity, leading to high mutation rates. This characteristic eventually leads to a wide range of new variants that resist current antiviral drugs targeting viral proteins, such as amantadine for IAV matrix-2 (M2) ion channels and oseltamivir for IAV neuraminidase. This situation highlights a critical need to develop broad-spectrum, host-directed antiviral drugs that are less prone to resistance and effective against both existing and emerging viral infections.

Nuclear receptors (NRs) are members of a large family of transcription factors that coordinate and regulate a wide range of genes in response to hormonal, metabolic, developmental, and environmental signals. Several viruses are known to exploit NRs to control gene expression or to optimize the cellular environment to support the viral life cycle. Estrogen-related receptors (ERRs) are orphan NRs because their specific endogenous ligands have not yet been identified. In mammals, there are three isoforms (ERRα, ERRβ, and ERRy, encoded by *Esrra*, *Esrrb*, *and Esrrg*, respectively), with ERRβ primarily functioning in mice to maintain pluripotency in embryonic stem cells, though not exclusively. In contrast, ERRα and ERRy are widely expressed across multiple organs and regulate many similar gene programs, including energy metabolism and bone homeostasis. It has been observed that ERRy expression is highly inducible and dynamically regulated by membrane receptors that respond to diverse signals. Therefore, ERRy is considered a crucial downstream mediator of various endocrine and metabolic signals. Infection with *Salmonella* enterica serovar *Typhimurium* (*S. typhimurium*), an intramacrophage bacterium, results in increased hepatic ERRy expression, which subsequently activates hepatic hepcidin, a key hormone that regulates iron levels, leading to alterations in host iron homeostasis. Furthermore, an inverse agonist of ERRy notably improved host survival following infection with multidrug-resistant *S. typhimurium*, indicating that ERRy is a potential host target for controlling S. typhimurium infection.

Since lipids and lipid droplets (LDs) play a crucial role in supporting the life cycle of many diverse viruses at all stages, including viral intracellular trafficking, replication, and egress, lipid metabolism is an emerging potential target for antiviral strategies. Interestingly, recent reports demonstrate that LDs accumulated early after viral infection are necessary for an effective interferon response, but at later stages, accumulated LDs are broken down by LD-associated lipases. The released lipids, especially free fatty acids (FFAs), are used to form viral replication compartments, assemble viral particles, or provide energy for viral replication. During the replication of various viruses, sterol regulatory element-binding proteins (SREBPs, encoded by Srebp1) activate genes involved in fatty acid and cholesterol biosynthesis and LD formation. However, how the SREBPs are regulated in virus-infected cells remains unclear.

### [PRIOR ART DOCUMENT]

### [Non-patent literature]

Baek, Y. B. et al. Therapeutic strategy targeting host lipolysis limits infection by SARS-CoV-2 and influenza A virus. Signal Transduct. Target. Ther. 7, 367 (2022).

### DISCLOSURE OF THE INVENTION

### PROBLEMS TO BE SOLVED BY THE INVENTION

The present disclosure provides an antiviral pharmaceutical composition including an estrogen-related receptor γ (ERRy) inhibitor as an active ingredient.

However, problems to be solved by the present disclosure are not limited to the above-described problems, and although not described herein, other problems to be solved by the present disclosure can be clearly understood by those skilled in the art from the following descriptions.

### MEANS FOR SOLVING THE PROBLEMS

An aspect of the present disclosure provides an antiviral pharmaceutical composition including an estrogen-related receptor γ (ERRy) inhibitor as an active ingredient.

### EFFECTS OF THE INVENTION

According to embodiments of the present disclosure, an antiviral pharmaceutical composition including an ERRy inhibitor as an active ingredient has antiviral effects. In particular, the antiviral pharmaceutical composition is effective in preventing or treating RNA virus infections. Specifically, the ERRy inhibitor, especially an ERRy inverse agonist, may exhibit broad-spectrum antiviral effects by inhibiting ERRy activity in RNA virus-infected cells, thereby blocking fatty acid biosynthesis mediated by sterol regulatory element-binding protein 1c (SREBP1c).

### BRIEF DESCRIPTION OF THE DRAWINGS

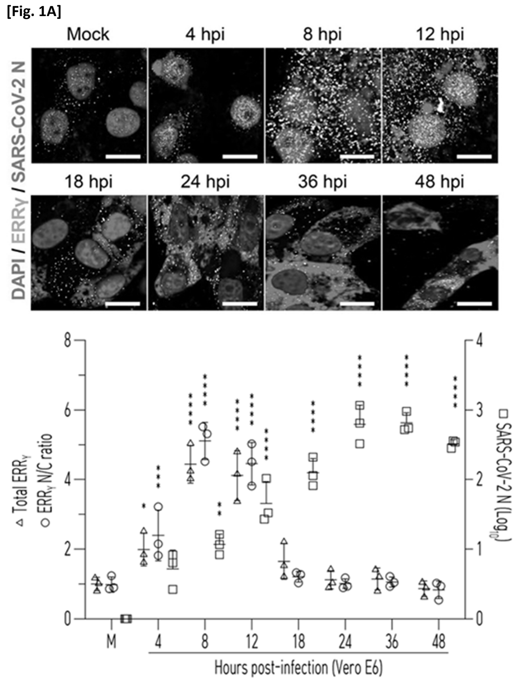

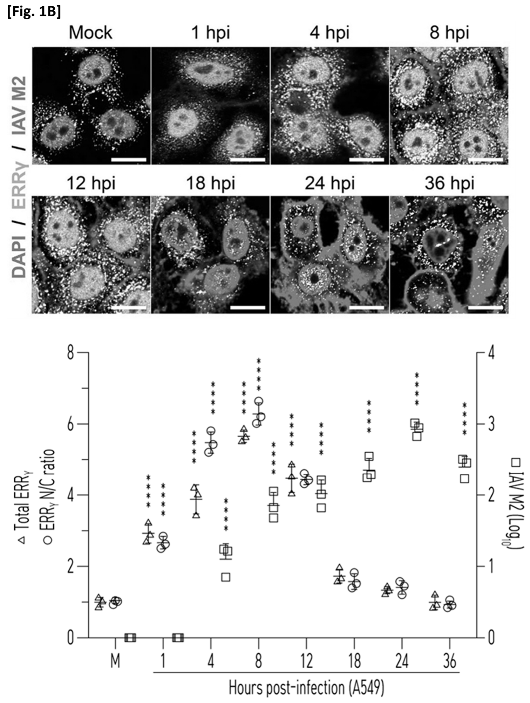

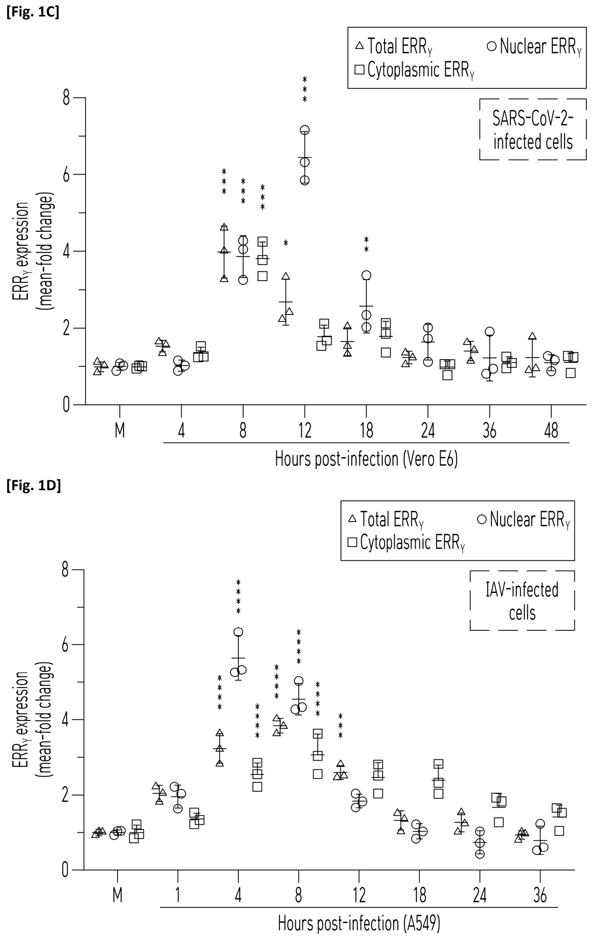

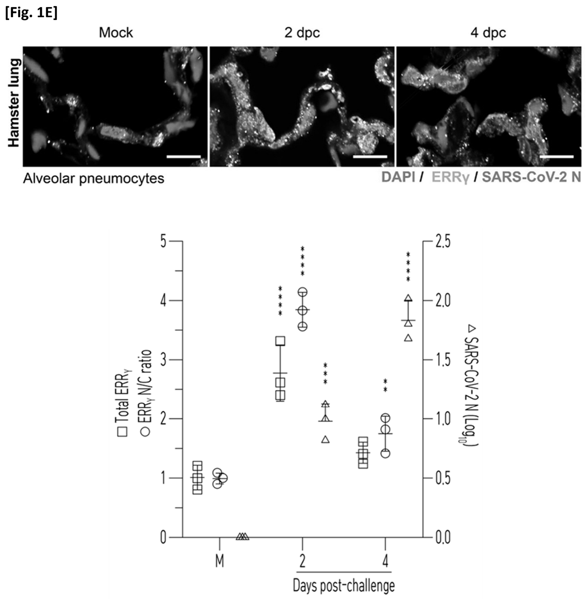

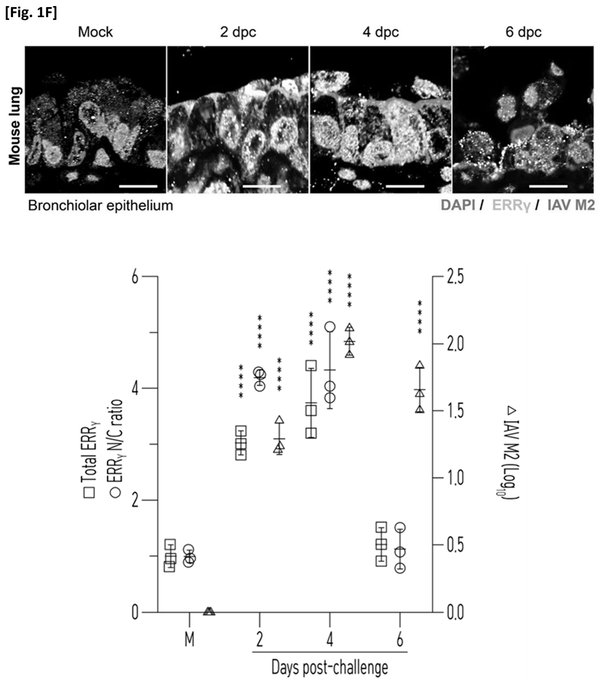

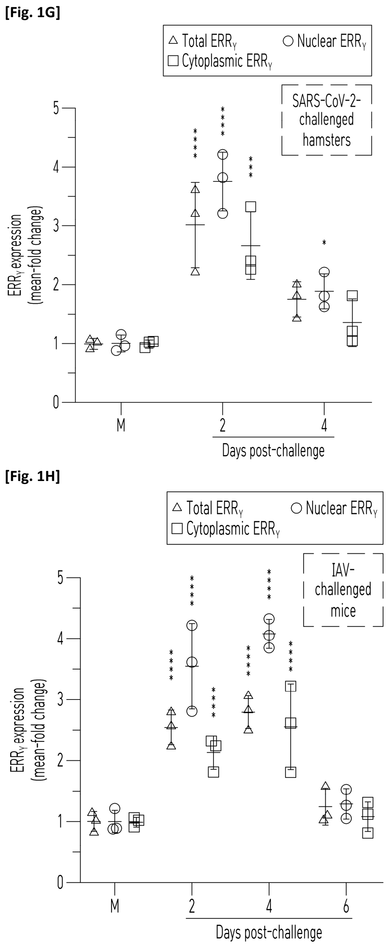

**FIG. 1A to FIG. 1H** are experimental results showing that in vitro and in vivo Infections with SARS-CoV-2 and IAV activate and translocate ERRy into the nucleus, in an example of the present disclosure. **(****FIGS. 1A****, 1B)** Representative confocal images (left) and quantification (right) of sequential changes of ERRy (white) and SARS-CoV-2 N protein (red) in the cytoplasm and nucleus of Vero E6 cells infected with SARS-CoV-2 KCDC03 strain at an MOI of 0.1 FFU (FIG. 1A) and of ERRy (white) and IAV M2 protein (red) in the cytoplasm and nucleus of A549 cells infected with IAV PR8 strain at an MOI of 1 FFU (FIG. 1B). **(****FIGS. 1C****,** **1D)** Quantification of ERRy in the cytoplasmic fraction, nuclear fraction, and whole-cell lysate of the Vero E6 cells infected with SARS-CoV-2 KCDC03 strain at an MOI of 0.1 FFU (FIG. 1C) and of the A549 cells infected with IAV PR8 strain at an MOI of 1 FFU (FIG. 1D) by western blot analysis. **(****FIGS. 1E****, 1F)** Representative confocal images (left) and quantification (right) of ERRy (white) and SARS-CoV-2 N protein (red) in pneumocytes of lungs sampled from the Syrian hamsters challenged with 10⁵ TCID₅₀ of SARS-CoV-2 KCDC03 strain (FIG. 1E) and of ERRy (white) and IAV M2 protein (red) in bronchiolar epithelial cells of lungs sampled from the mice challenged with 10³ PFU of mouse-adapted IAV PR8 strain (FIG. 1F). dpc: days post-challenge. **(****FIGS. 1G****,** **1H)** Quantification of ERRy in the cytoplasmic fraction, nuclear fraction, and whole cell lysate of the lungs from the Syrian hamsters challenged with 10⁵ TCID₅₀ of SARS-CoV-2 KCDC03 strain (FIG. 1G) and in the lungs from the mice challenged with 10³ PFU of mouse-adapted IAV PR8 strain (FIG. 1H) by western blot analysis.

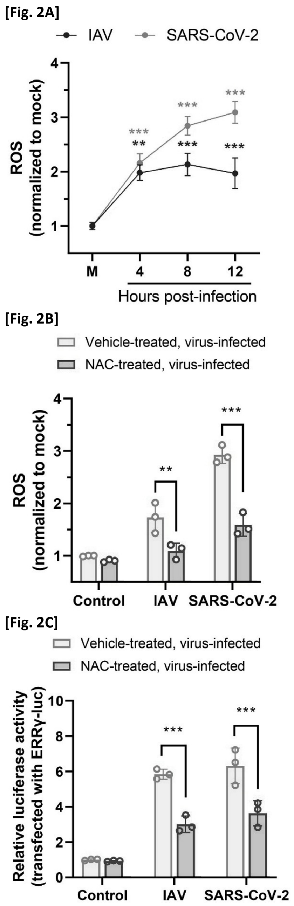

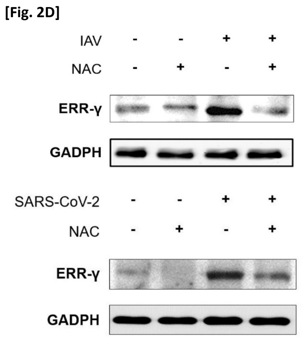

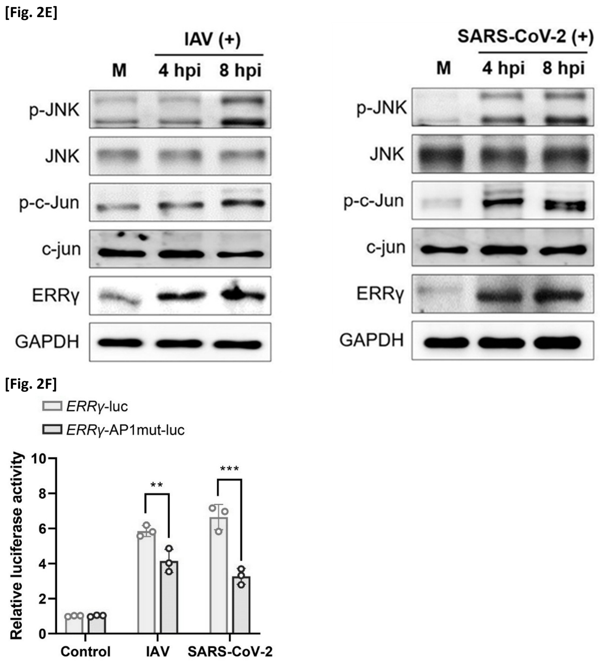

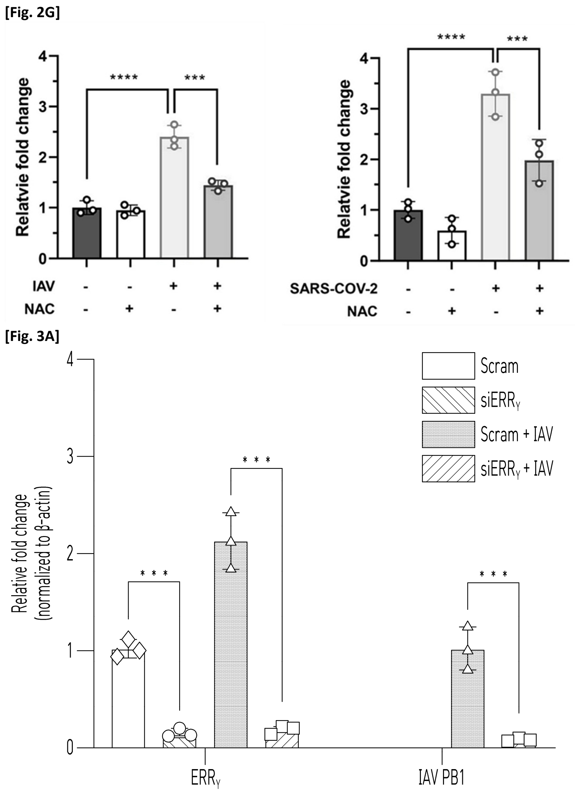

**FIG. 2A to FIG. 2G** are experimental results showing that SARS-CoV-2 and IAV induce and transactivate ERRy through a ROS-induced JNK/c-Jun pathway, in an example of the present disclosure. **(****FIG. 2A****)** ROS levels in SARS-CoV-2-infected Vero E6 cells (MOI = 0.1 FFU) and IAV-infected A549 cells (MOI = 1 FFU) at different time points, which are normalized to mock-infected control. **(****FIG. 2B****)** Reduction in the ROS levels in IAV-infected A549 cells or SARS-CoV-2-infected Vero E6 cells by treatment with N-acetylcysteine (NAC) antioxidant. **(****FIG. 2C****)** Reduction of the luciferase activity by treatment with NAC in IAV-infected A549 cells or SARS-CoV-2-infected Vero E6 cells harboring a plasmid with the full-length *ERRγ* promoter-luciferase (*ERRγ*-luc)*.* **(****FIG. 2D****)** Reduction in ERRγ expression by treatment with NAC in IAV- and SARS-CoV-2-infected cells. GAPDH was used as a loading control. **(****FIG. 2E****)** Phosphorylation of JNK and c-Jun in response to infection with either IAV or SARS-CoV-2. GAPDH was used as a loading control. **(****FIG. 2F****)** Lesser activation of site-specific AP1 mutant *ERRγ* promoter (*ERRγ*-AP1mut-luc) than full-length *ERRγ* promoter (*ERRγ*-luc) by infection with IAV and SARS-CoV-2. **(****FIG. 2G****)** Chromatin immunoprecipitation assay. Marked reduction in IAV- and SARS-CoV-2-mediated occupancy of c-Jun on the AP1 regulatory element site of *ERRγ* promoter by treatment with NAC.

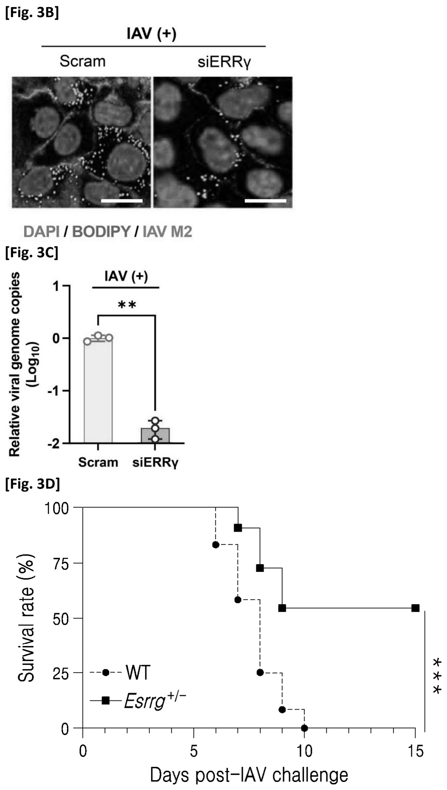

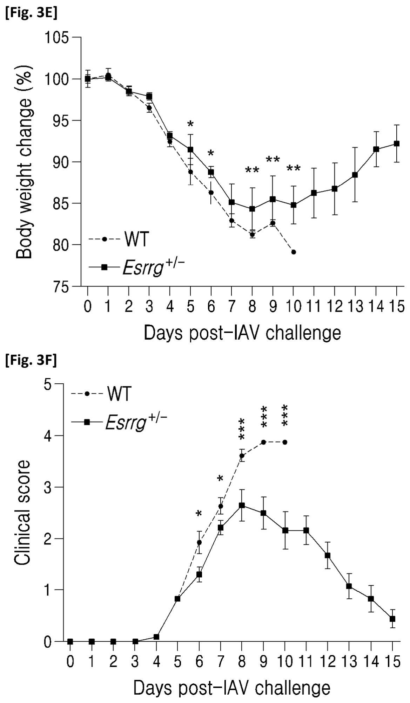

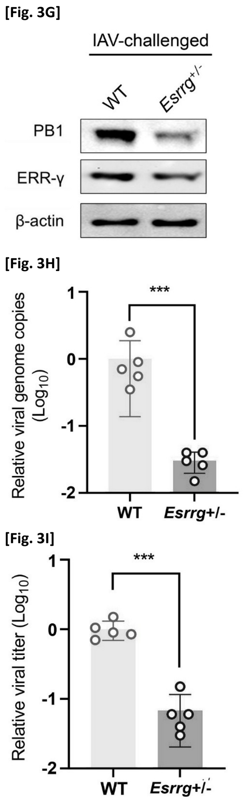

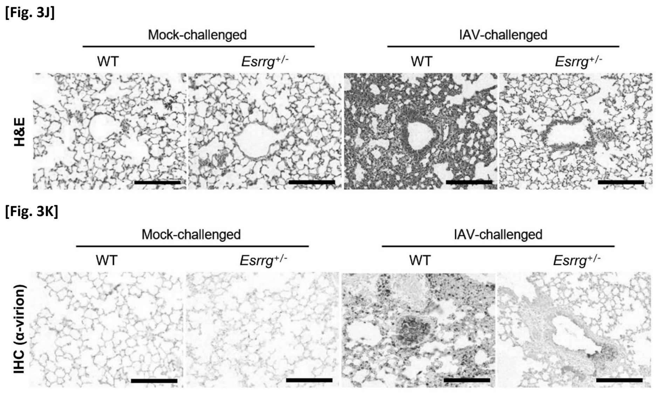

**FIG. 3A to FIG. 3K** are experimental results showing that deficiency of ERRy inhibits IAV replication in vitro and in vivo, in an example of the present disclosure. **(****FIG. 3A****)** A graphical representation of western blot results. Compared to scrambled siRNA transfection control, knockdown of ERRy by the transfection with siRNA against ERRy reduced expression levels of ERRy and IAV PB1 protein in the A549 cells infected with IAV PR8 strain at an MOI of 1 FFU. **(****FIG. 3B****)** Representative confocal images of IAV M2 protein (red) and BODIPY-stained intracellular LDs (green) in A549 cells infected with IAV PR8 strain at an MOI of 1 FFU after ERRy knockdown. **(****FIG. 3C****)** Reduction in IAV viral genome copy number by ERRy knockdown. **(****FIGS. 3D-3F****)** Comparison of survival rates (FIG. 3D), body weight change (FIG. 3E), and clinical score (FIG. 3F) between wild type (WT) and *Esrrg*^{*+*/*-*} mice after challenge with 10³ PFU of mouse-adapted IAV PR8 strain (n = 16). **(****FIGS. 3G-3I****)** Comparison of levels of IAV PB1 protein (FIG. 3G), viral genome copy (FIG. 3H), and viral titer (FIG. 3I) in the lungs from wild type (WT) and *Esrrg*^{+/-} mice after challenge with 10³ PFU of mouse-adapted IAV PR8 strain (n = 5). **(****FIGS. 3J****,** **3K)** Representative images of histological lesion changes (FIG. 3J) and immunohistochemical changes of viral antigen distribution (FIG. 3K) in the lungs from the WT or *Esrrg*^{+/-} mice after challenge with 10³ PFU of the mouse-adapted IAV PR8 strain.

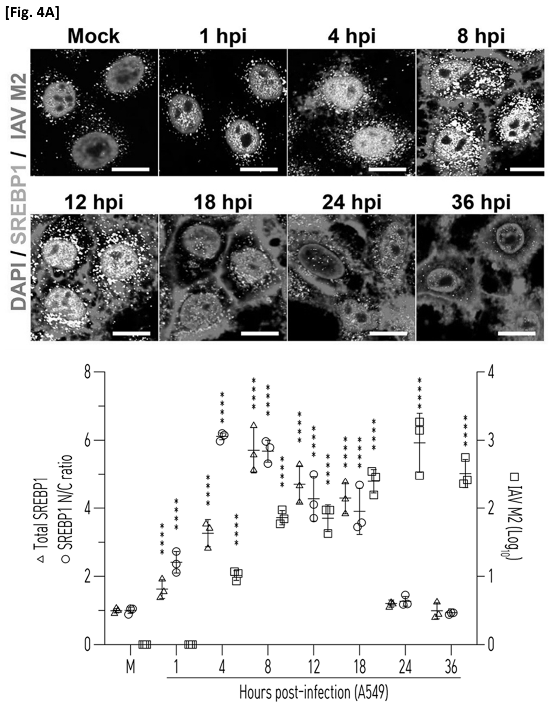

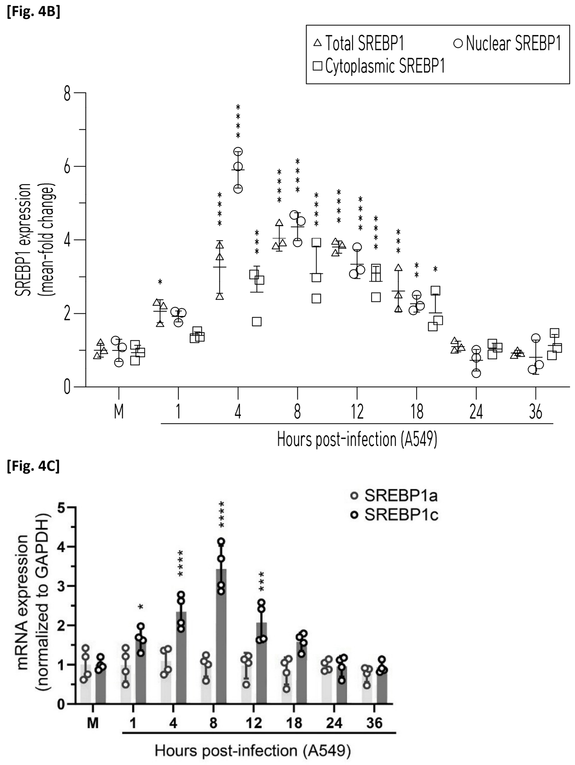

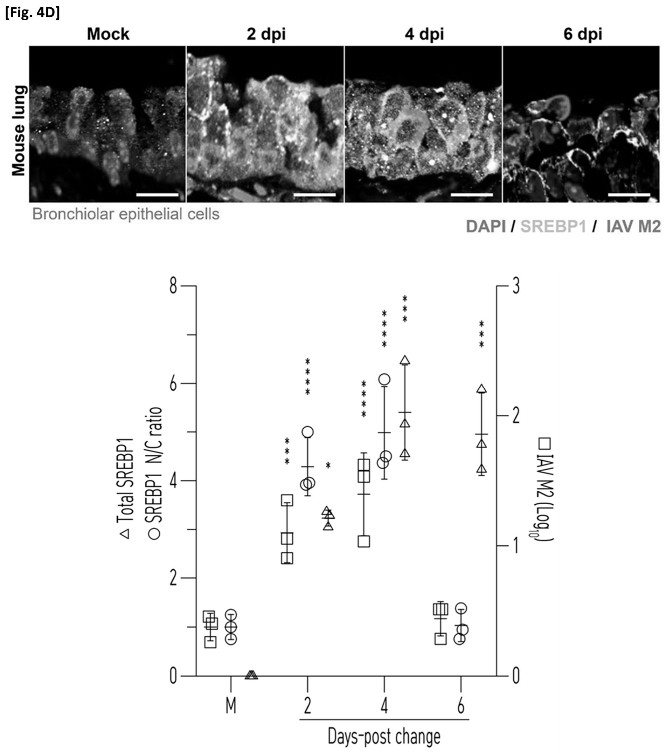

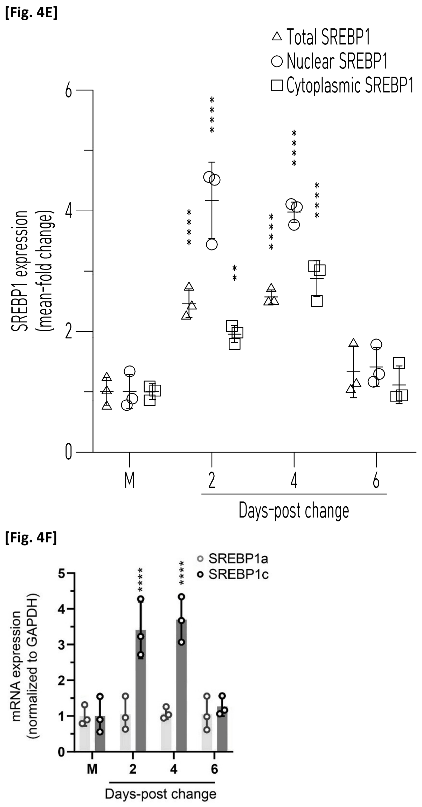

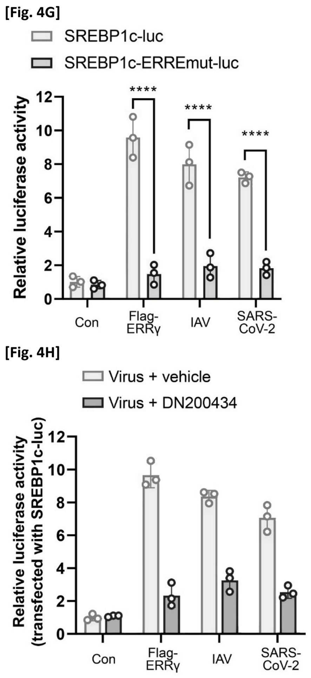

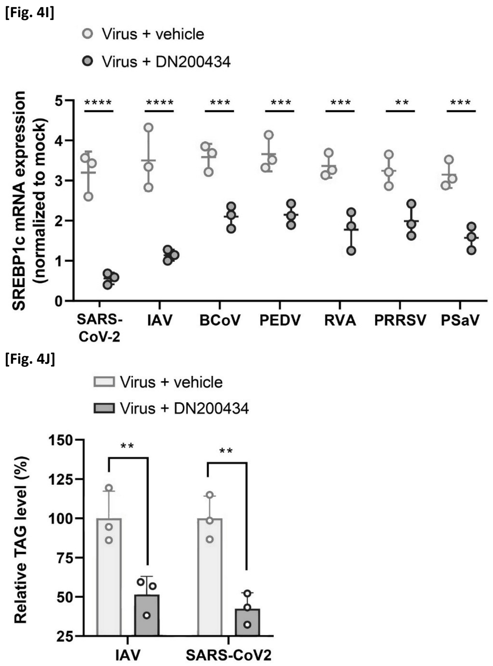

**FIG. 4A to FIG. 4J** are experimental results showing that virus-induced ERRy transactivates SREBP1c, in an example of the present disclosure. **(****FIG. 4A****)** Representative confocal images (left) and quantification (right) of SREBP1 (white) and IAV M2 protein (red) in the cytoplasm and nucleus of A549 cells infected with the IAV PR8 strain at an MOI of 1 FFU. Scale bars = 25 µm. **(****FIG. 4B****)** Quantification of SREBP1 protein in the cytoplasmic fraction, nuclear fraction, and whole cell lysate of A549 cells infected with the IAV PR8 strain at an MOI of 1 FFU, analyzed by western blot. **(****FIG. 4C****)** Quantification of transcription levels of SREBP1a and SREBP1c genes in A549 cells infected with IAV PR8 strain at an MOI of 1 FFU using RT-qPCR analysis. **(****FIG. 4D****)** Representative confocal images (left) and quantification (right) of SREBP1 (white) and IAV M2 protein (red) in the cytoplasm and nucleus of bronchiolar epithelial cells from lungs sampled from mice challenged with 10³ PFU of mouse-adapted IAV PR8 strain. Scale bars = 25 µm. **(****FIG. 4E****)** Quantification of SREBP1 in the cytoplasmic fraction, nuclear fraction, and whole-cell lysate of the lungs sampled from the mice challenged with 10³ PFU of mouse-adapted IAV PR8 strain by western blot analysis. **(****FIG. 4F****)** Quantification of transcription levels of Srebp1a and Srebp1c genes in the lungs of the mice challenged with 10³ PFU of mouse-adapted IAV PR8 strain by RT-qPCR analysis. **(****FIG. 4G****)** ERRE-dependent activation of the SREBP1c promoter in IAV- or SARS-CoV-2-infected cells. Cells transfected with either SREBP1c-luc or SREBP1c-ERREmut-luc plasmid are left mock-infected, transfected with pcDNA3.0-Flag-ERRy (encoding Flag-ERRy), or infected with IAV at an MOI of 2 FFU or SARS-CoV-2 at an MOI of 2 FFU and then treated with DN200434 at 20 µM. **(****FIG. 4H****)** Treatment with DN200434, an inverse agonist of ERRy, inhibits ERRE-dependent activation of the SREBP1c promoter in IAV- or SARS-CoV-2-infected cells. Cells transfected with SREBP1c-luc are left mock-infected, transfected with Flag-ERRγ, or infected with either IAV (MOI = 2 FFU) or SARS-CoV-2 (MOI = 2 FFU), and then treated with DN200434 at 20 µM. **(****FIG. 4I****)** Blockade of ERRy by the treatment with its inverse agonist DN200434 at 20 µM inhibits transcriptional activity of its target SREBP1c gene in cells infected individually with each target virus. **(****FIG. 4J****)** Inhibition of ERRy by treatment with 20 µM DN200434 reduces triacylglyceride formation in cells compared to mock-treated controls.

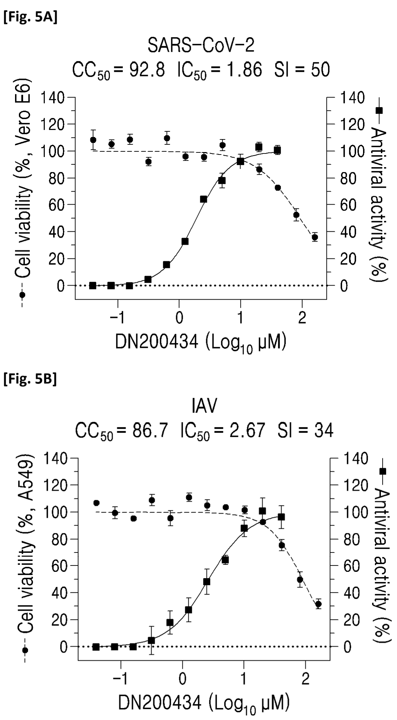

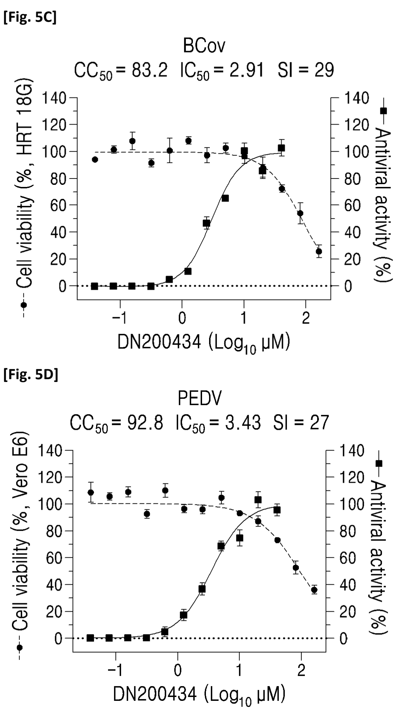

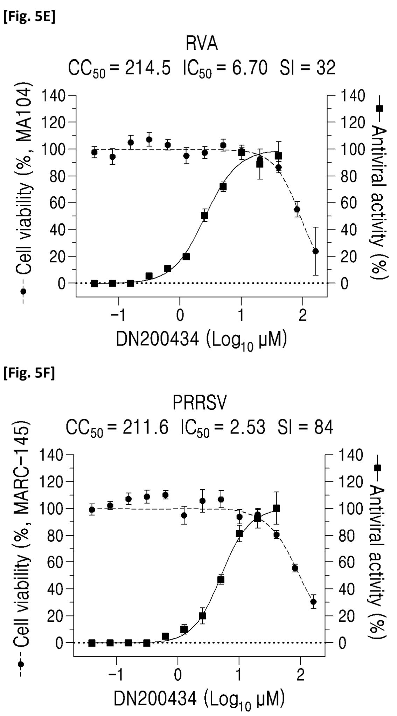

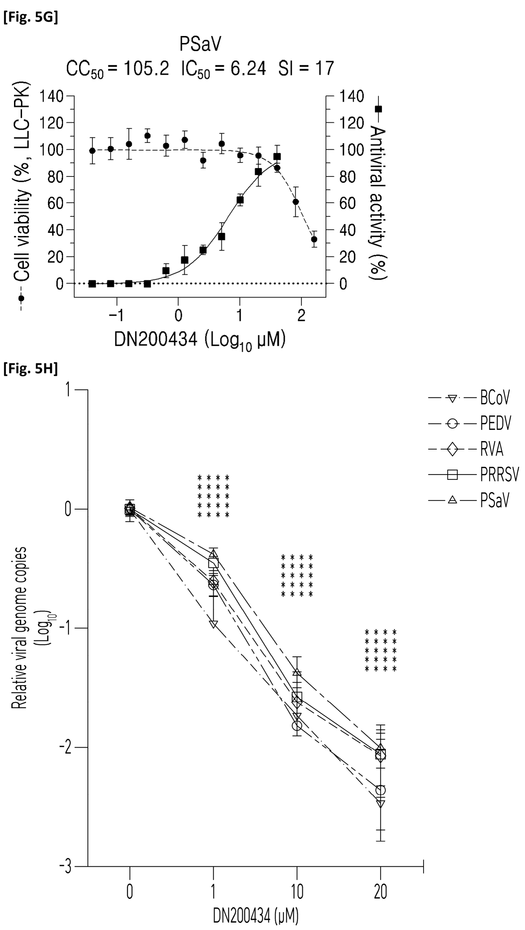

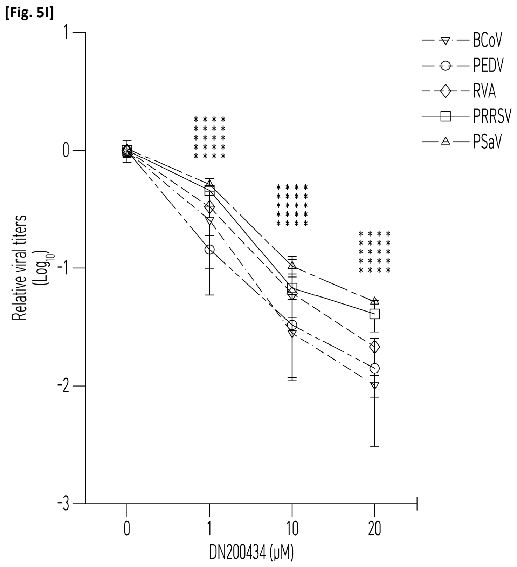

**FIG. 5A** to **FIG. 5G** show, in an example of the present disclosure, dose-response curves for half maximal inhibitory concentration (IC₅₀) and half maximal cytotoxicity concentration (CC₅₀), and resultant selective index (SI) of DN200434 against seven target RNA viruses: SARS-CoV-2 KCDC03 (MOI = 0.1), influenza A virus (IAV) PR8 strain (MOI = 1 FFU), BCoV KWD strain (MOI = 1 FFU), PEDV QIAP1401 strain (MOI = 0.1 FFU), RVA NCDV strain (MOI = 0.1 FFU), PRRSV LMY strain (MOI = 0.1 FFU), and PSaV Cowden strain (MOI = 0.1 FFU). The viral yield in the cell supernatant was quantified using a cell culture immunofluorescence assay. Cytotoxicity of DN200434 in each cell line was measured using an MTT assay. **FIG. 5H** and **FIG. 5I** show, in an example of the present disclosure, effect of DN200434 on reduction in viral genome copy numbers (FIG. 5H) and viral infectivity titers (FIG. 5I) for five different RNA viruses in infected cells.

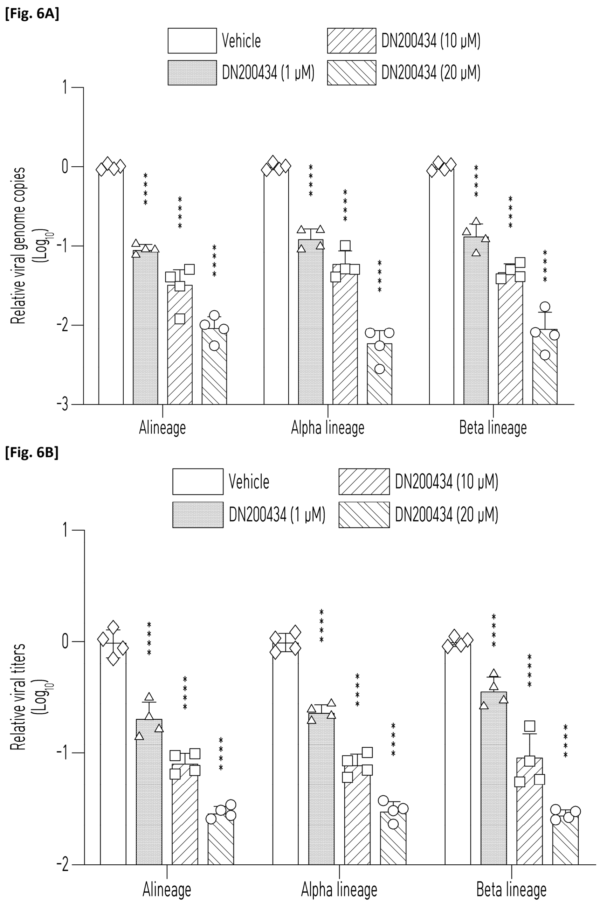

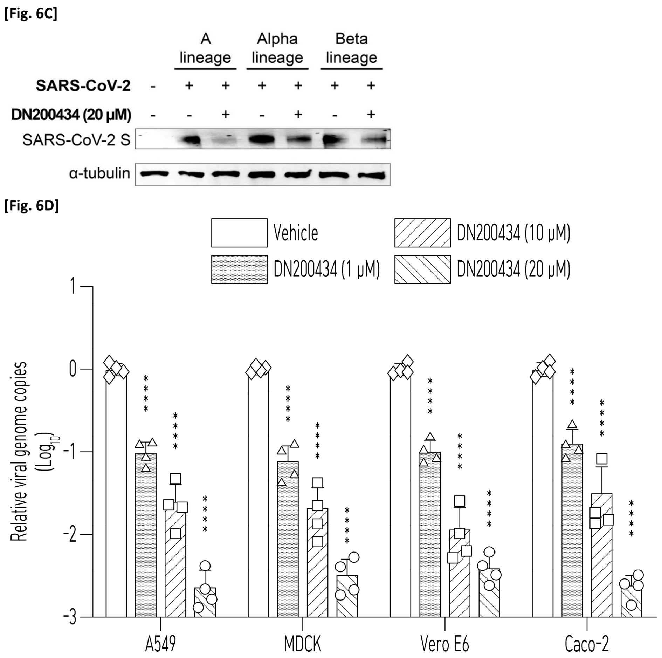

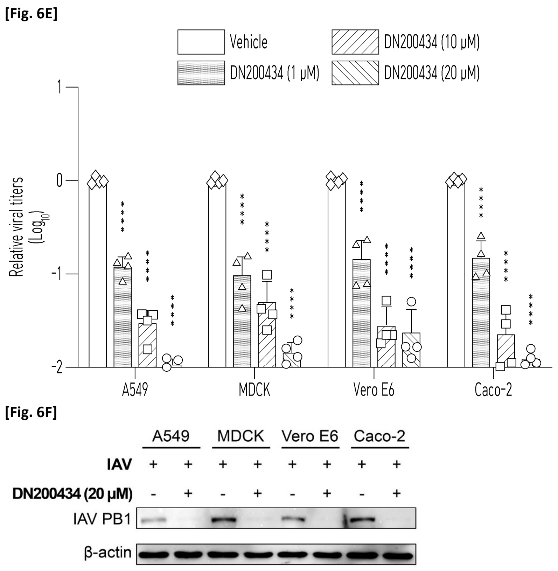

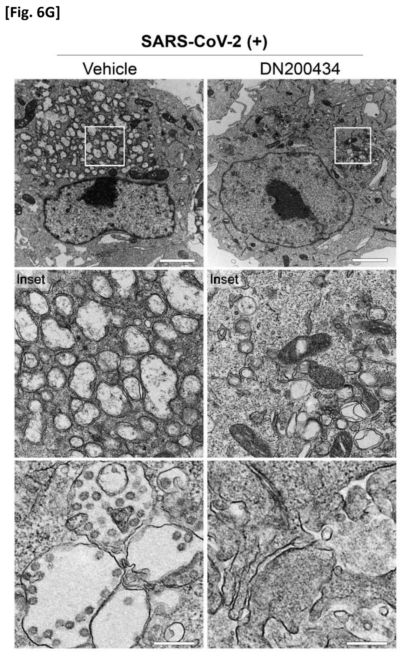

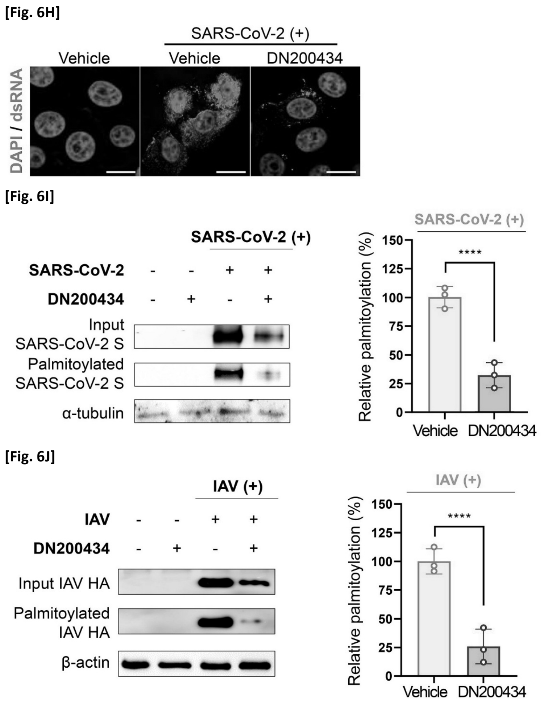

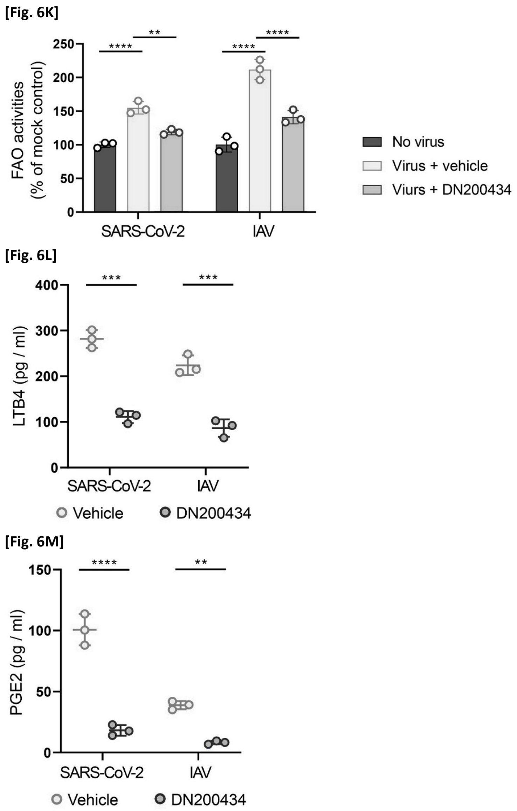

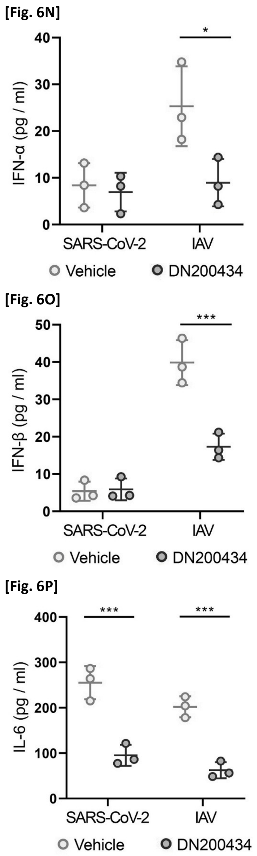

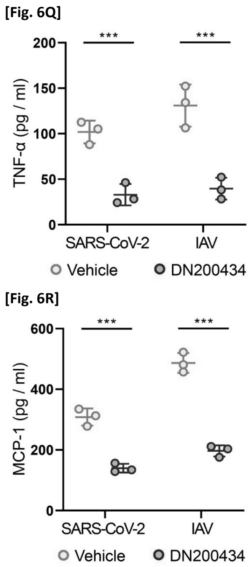

**FIG. 6A** to **FIG. 6R** are experimental results showing the in vitro antiviral and anti-inflammatory cytokine effects of the ERRy inverse agonist (DN200434), in an example of the present disclosure. **(****FIGS. 6A-6C****)** Dose-dependent reduction in the genomic replication numbers (FIG. 6A), infectious progeny production (FIG. 6B), and protein synthesis (FIG. 6C) upon treatment with DN200434 in the Vero E6 cells (MOI = 0.1 FFU) infected with SARS-CoV-2 KCDC03 (closely related to early ChineseA-lineage viruses), KDCA51463 (closely related to alpha lineage viruses including the UK variants), and KDCA55905 (closely related to beta lineage viruses with South African variants) strains. **(****FIGS. 6D-6F****)** Dose-dependent reduction in the genomic replication numbers (FIG. 6D), infectious progeny production (FIG. 6E), and protein synthesis (FIG. 6F) in A549, MDCK, Vero E6, and Caco-2 cells infected with IAV PR8 (MOI = 0.1 FFU). (FIG. 6G) Inhibition of double-membrane vesicle (DMV) formation in the SARS-CoV-2-infected cells (MOI = 0.1 FFU) by the treatment with DN200434. Note relatively few perinuclear DMVs (upper and middle panels) or virus particles (lower panel) in the DN200434-treated cells compared to vehicle-treated control cells. Scale bars: upper panels, 1 um; lower panels, 800 nm. (FIG. 6H) Inhibition of double-stranded RNA (dsRNA) formation in the SARS-CoV-2-infected cells by the treatment with DN200434. Note the relatively small amount of dsRNA-positive fluorescence signals in the DN200434-treated cells compared to vehicle-treated control cells. Scale bars = 40 µm. **(****FIGS. 6I****,** **6J)** Representative western blot (left) and quantification (right) of inhibitory effects of DN200434 on palmitoylations of S protein in SARS-CoV-2-infected cells (MOI = 0.1 FFU) and HA protein in IAV-infected cells (MOI = 1 FFU) at 24 hpi, respectively. (FIG. 6K) Graphical representation of inhibitory effects of DN200434 on intracellular fatty acid oxidation (FAO) activities in the SARS-CoV-2-infected cells (MOI = 0.1 FFU) and IAV-infected cells (MOI = 1 FFU) at 36 hpi and 24 hpi, respectively. **(****FIGS. 6L-6R****)** The graphical representation of the LTB4, PGE2, IFN-α, IFN-β, IL-6, TNF-α, and MCP-1 levels in SARS-CoV-2-infected Vero E6 cells (MOI = 0.1 FFU) and IAV-infected A549 cells (MOI = 1 FFU), which were vehicle-treated or treated with DN200434 (Levels of each eicosanoide and cytokine were analyzed by ELISA).

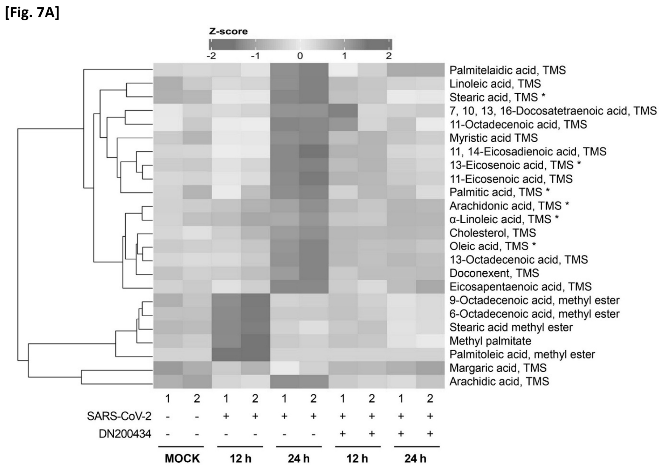

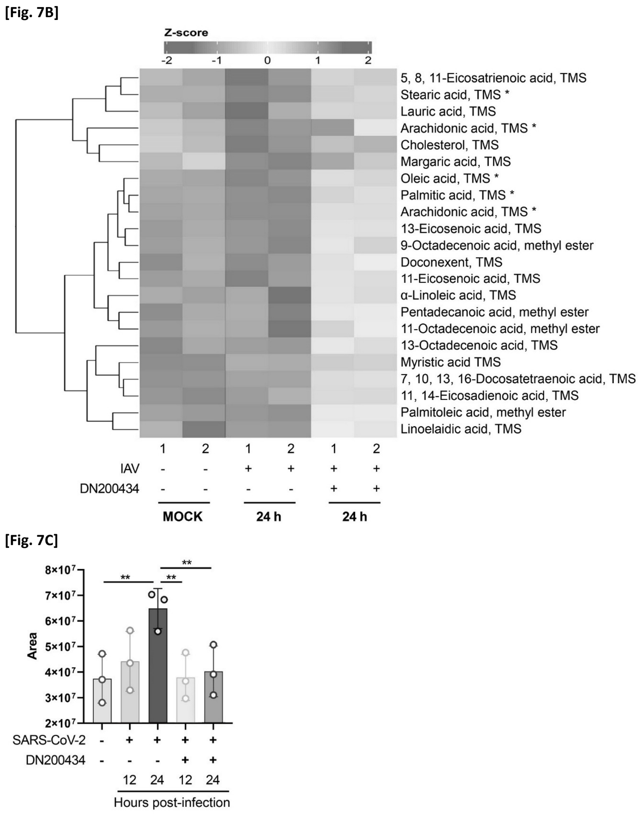

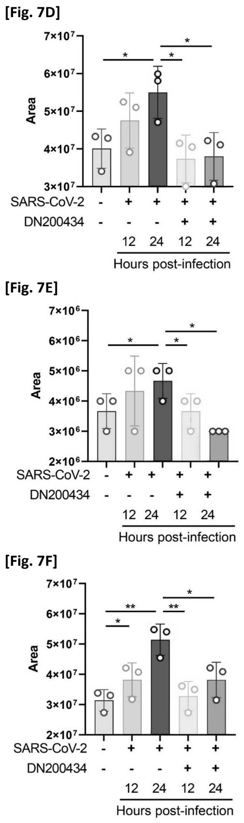

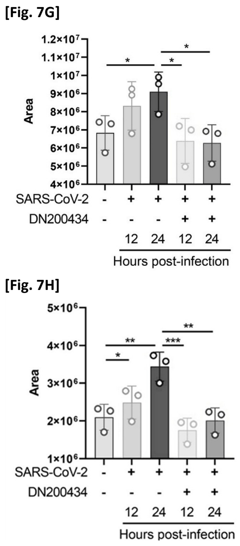

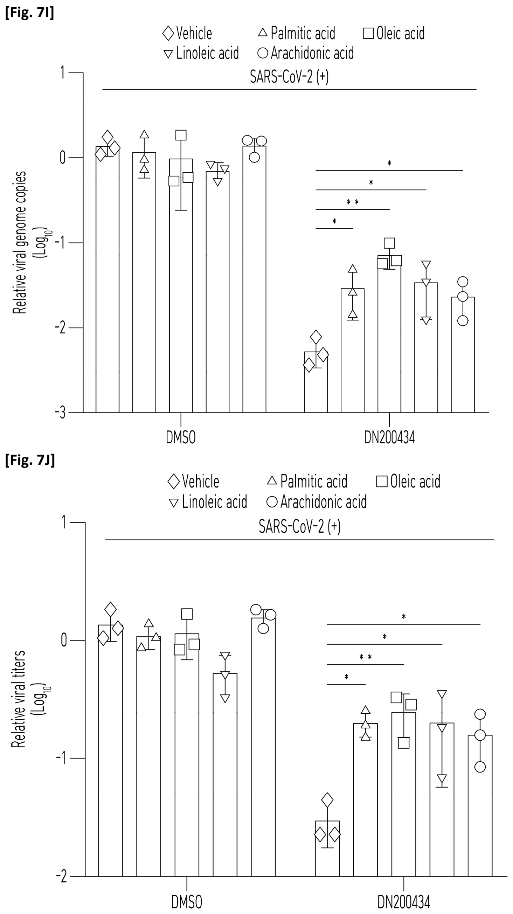

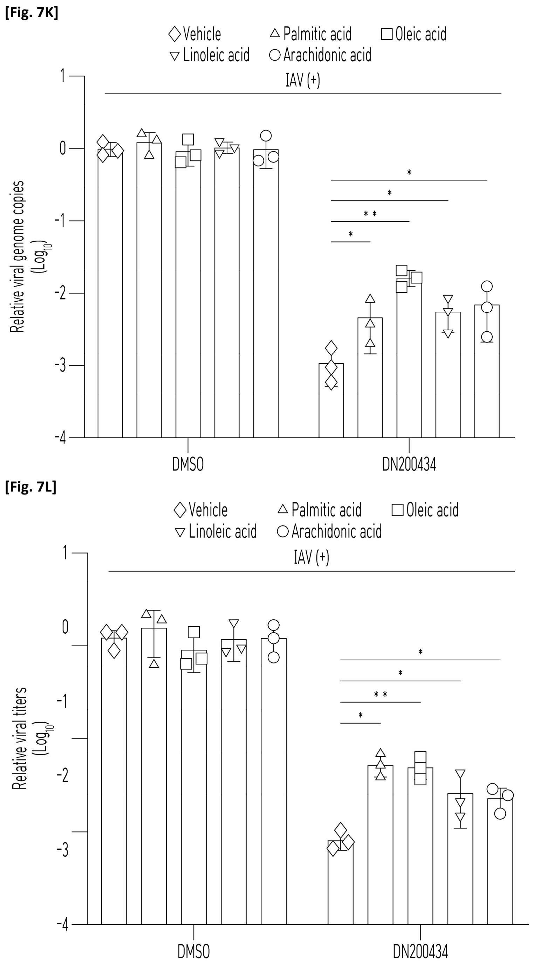

**FIG. 7A to FIG. 7L** are experimental results showing that free fatty acid profile and restoration of suppressed viral replication by adding exogenous fatty acids, in an example of the present disclosure. **(****FIGS. 7A****, 7B)** Heatmaps showing the changes in fatty acids following treatment of SARS-CoV-2-infected Vero E6 cells (MOI = 0.1 FFU) and IAV-infected A549 cells (MOI = 1 FFU) with DN200434. The cells were mock-infected or infected with the above virus and treated with vehicle or 20 µM of DN200434. The incubation time differed between the SARS-CoV-2 and IAV experiments: 12 h and 24 h, respectively, for the SARS-CoV-2 experiment, and 36 h for the IAV experiment. A higher intensity of fatty acid in the rectangle than normal is indicated in red, and a lower is indicated in blue. TMS: trimethylsilyl ester. *: Representative fatty acids for further analysis. **(****FIGS. 7C-7H****)** DN200434 inhibited the synthesis of representative fatty acids induced by SARS-CoV-2 infection. Palmitic acid (FIG. 7C), oleic acid (FIG. 7D), α-linoleic acid (FIG. 7E), stearic acid (FIG. 7F), arachidonic acid (FIG. 7G), and 13-eicosenoic acid (FIG. 7H). **(****FIGS. 7I-7L****)** Increase in viral genome copy numbers and progeny numbers of SARS-CoV-2 (FIGS. 7I, 7J) and IAV (FIGS. 7K, 7L) through individual supplementation of exogenous palmitic, oleic, linoleic, or arachidonic acids in the above FFA-deprived condition.

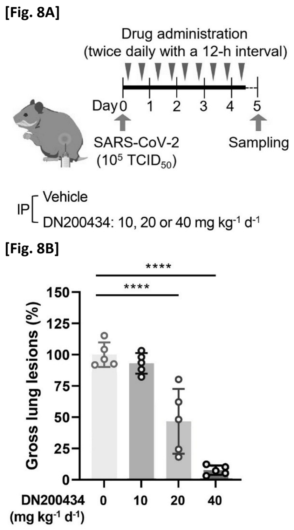

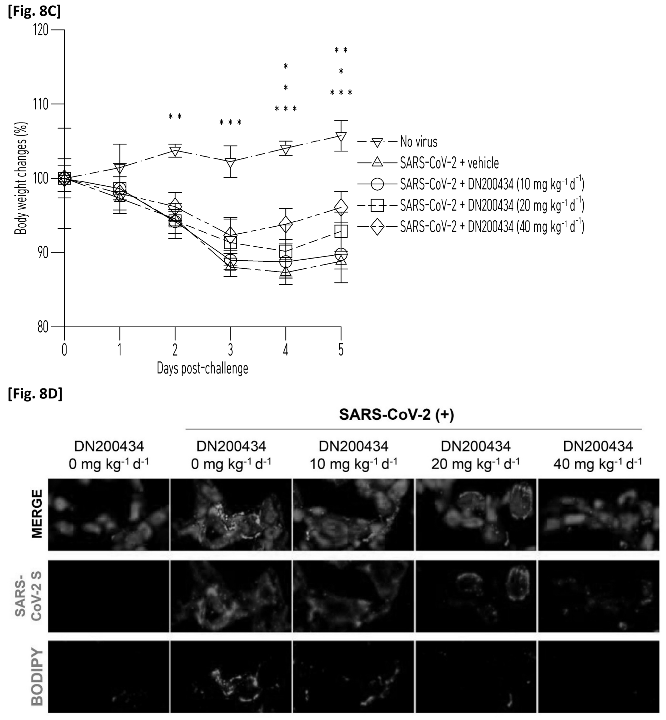

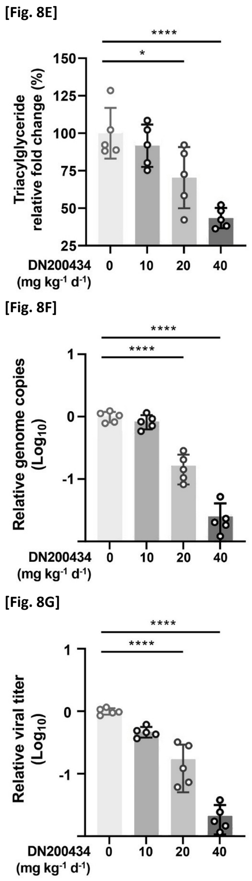

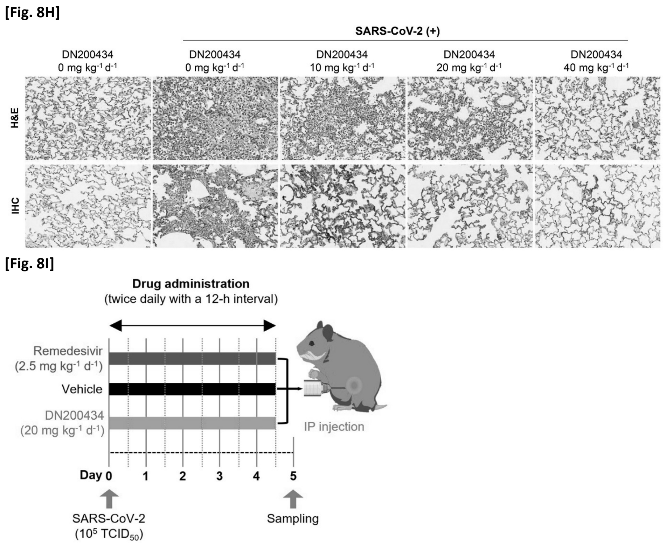

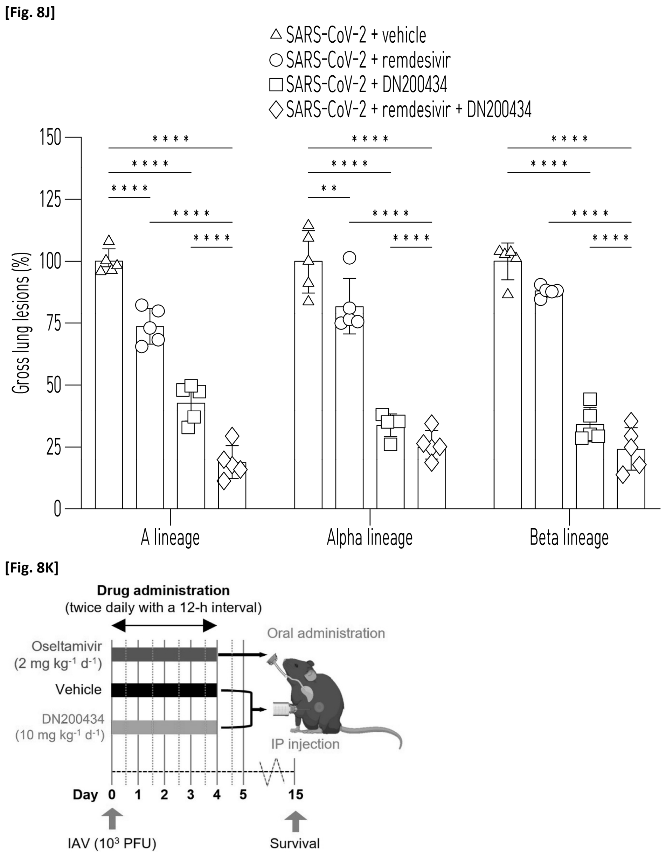

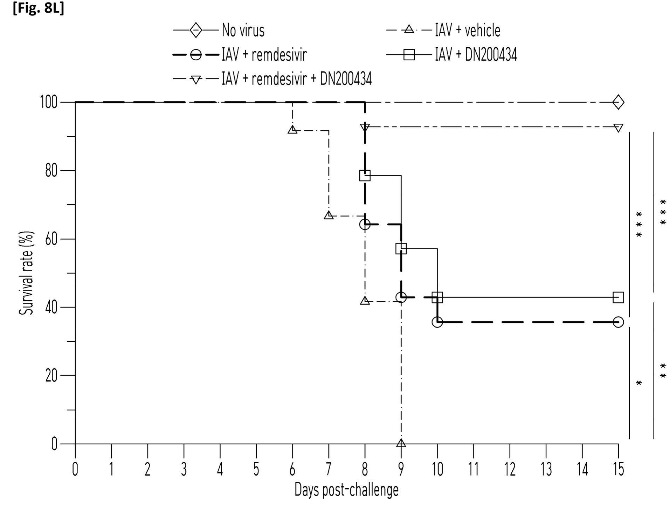

**FIG. 8A to FIG. 8L** are experimental results showing the in vivo antiviral effects of the ERRy inverse agonist (DN200434) against SARS-CoV-2 and influenza A virus (IAV), as an example of the present disclosure. **(****FIG. 8A****)** Scheme of chemical administration twice daily for four and a half consecutive days after challenge with 10⁵ TCID₅₀ of SARS-CoV-2 KCDC03 strain to Syrian hamsters (*n =* 5). **(****FIG. 8B****)** Reduction in SARS-CoV-2-induced gross lung lesions in hamsters by treatment with DN200434. **(****FIG. 8C****)** Effect of treatments with DN200434 on the recovery of body weight in each group. **(****FIG. 8D****)** Representative images of dose-dependent reduction in BODIPY-stained LDs (green, lower panels) and inhibition of SARS-CoV-2 replication (red, middle panels) in alveolar pneumocytes of lung tissues sampled from SARS-CoV-2-challenged hamsters by treatment with DN200434. **(****FIGS. 8E-8G****)** Graphical representation of dose-dependent reduction in triacylglyceride (FIG. 8E), viral genome copy numbers (FIG. 8F), and infectious progeny virus titers (FIG. 8G) in lungs sampled from SARS-CoV-2-challenged hamsters (n = 5) by treatment with DN200434. **(****FIG. 8H****)** Representative images of histological lesion changes (upper panels) and immunohistochemical SARS-CoV-2-antigen distribution (lower panels) in the lungs. **(****FIG. 8I****)** Scheme of chemical administration of DN200434 and remdesivir, either individually or in combination, twice daily for four and a half consecutive days after challenge with 10⁵ TCID₅₀ of SARS-CoV-2 KCDC03 (closely related to early Chinese strains), KDCA51463 (Alpha lineage with British variants), and KDCA55905 (Beta lineage with South African variants) to Syrian hamsters (n = 5). **(****FIG. 8J****)** Reduction in gross lung lesions in Syrian hamsters challenged with each strain by combination therapy with DN200434 and remdesivir. **(****FIG. 8K****)** Scheme of chemical administration of DN200434 and oseltamivir, either individually or in combination, twice daily for four consecutive days after challenge with 10³ PFU of mouse-adapted IAV PR8 strain to mice (*n* = 16). **(****FIG. 8L****)** Survival rates (expressed as percentages) of IAV-challenged mice after treatment.

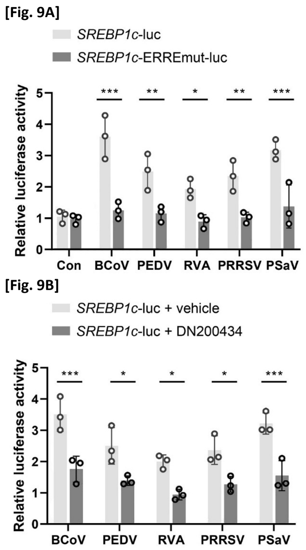

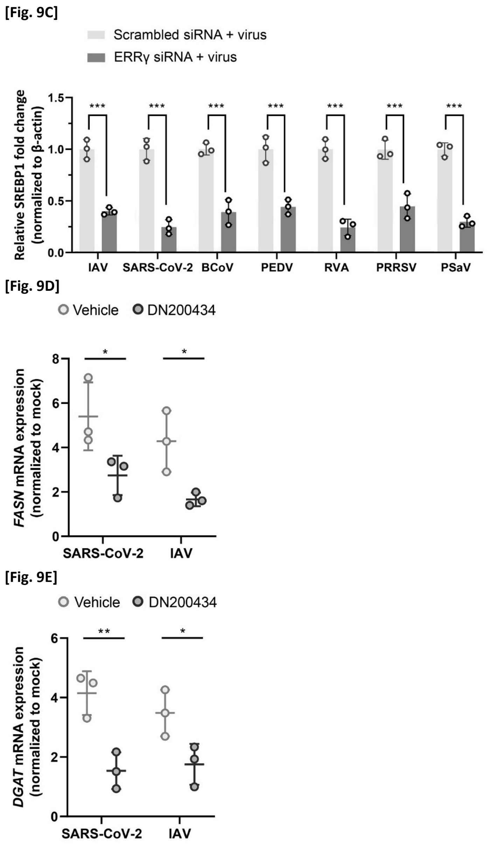

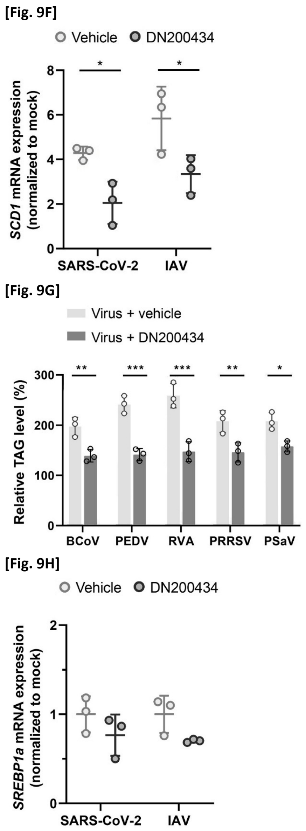

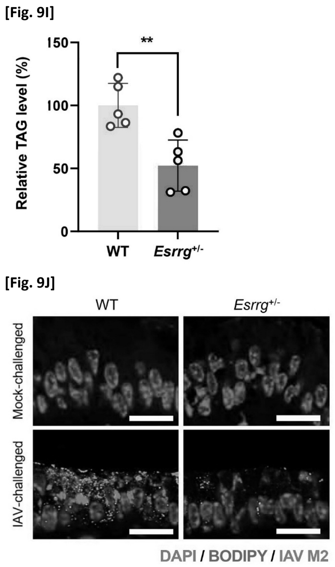

**FIG. 9A to FIG. 9J** are experimental results showing that virus-induced ERRy mediates SREBP1c-dependent fatty acid biosynthesis in virus-infected cells, in an example of the present disclosure. **(****FIG. 9A****)** An ERRE-dependent activation of the *SREBP1c* promoter in the virus-infected cells. Cells are transfected with either *SREBP1c*-luc or *SREBP1c*-ERREmut-luc plasmid and infected with BCoV KWD strain at an MOI of 0.1 FFU, PEDV QIAP1401 strain at an MOI of 0.1 FFU, RVA NCDV strain at an MOI of 0.1 FFU, PRRSV LMY strain at an MOI of 0.1 FFU, and PSaV Cowden strain at an MOI of 0.1 FFU after transfection with either *SREB1c*-luc or *SREBP1c*-ERREmut-luc plasmid. **(****FIG. 9B****)** Treatment with DN200434, an inverse agonist of ERRy, inhibits ERRE-dependent activation of *SREBP1c* promoter in virus-infected cells. Cells are transfected with *SREBP1c*-luc plasmid, infected with BCoV KWD strain (MOI = 0.1 FFU), PEDV QIAP1401 strain (MOI = 0.1 FFU), RVA NCDV strain (MOI = 0.1 FFU), PRRSV LMY strain (MOI = 0.1 FFU), and PSaV Cowden strain (MOI = 0.1 FFU) or left mock-infected, and then treated with DN200434 at 20 µM concentration. **(****FIG. 9C****)** Graphical representation of western blot results. Transfection with ERRy siRNA reduces SREBP1 protein levels in cells infected with each target virus individually. **(****FIGS. 9D-9F****)** RT-qPCR analysis of mRNA of genes involved in fatty acid biosynthesis. Inhibition of ERRy by DN200434 at 20 µM blocks mRNA expression of FASN (FIG. 9D), DGAT (FIG. 9E), and SCD1 (FIG. 9F) compared with vehicle-treated controls. **(****FIG. 9G****)** Inhibition of ERRy by treatment with DN200434 at a 20 µM concentration reduces the formation of TAG in the cells infected individually with BCoV KWD strain (MOI = 0.1 FFU), PEDV QIAP1401 strain (MOI = 0.1 FFU), RVA NCDV strain (MOI = 0.1 FFU), PRRSV LMY strain (MOI = 0.1 FFU), and PSaV Cowden strain (MOI = 0.1 FFU) compared to virus-infected, mock-treated controls. **(****FIG. 9H****)** RT-qPCR analysis of mRNA of the SREBP1a gene. Blockade of ERRy by the treatment with its inverse agonist DN200434 at a 20 µM concentration does not influence the transcriptional activity of the SREBP1α gene in cells infected with IAV (MOI = 1 FFU) or SARS-CoV-2 (MOI = 0.1 FFU). **(****FIG. 9I****)** Comparison of TAG levels in the lungs from wild type (WT) and *Esrrg*^{*+*/*-*} mice after challenge with 10³ PFU of mouse-adapted IAV PR8 strain (n = 5). **(****FIG. 9J****)** Representative confocal images of BODIPY-stained LDs and IAV M2 protein (red) in the bronchiolar epithelial cells of the lungs from the WT or *Esrrg*^{*+*/*-*} mice after challenge with 10³ PFU of mouse-adapted IAV PR8 strain. Scale bars = 25 µm.

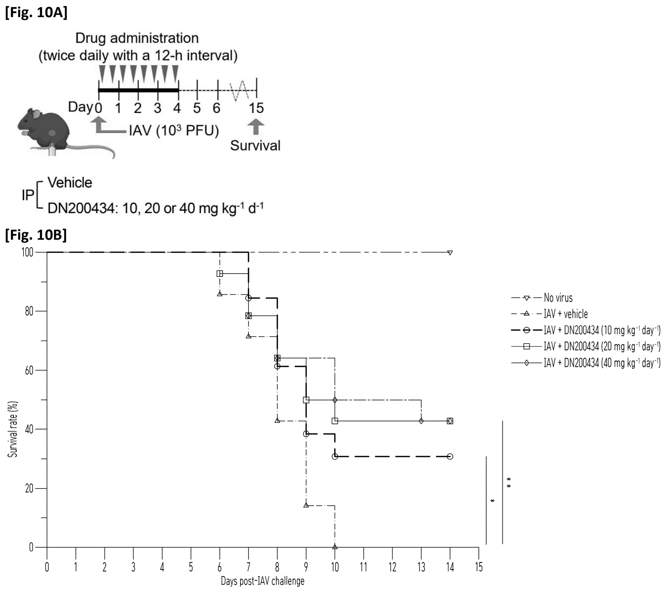

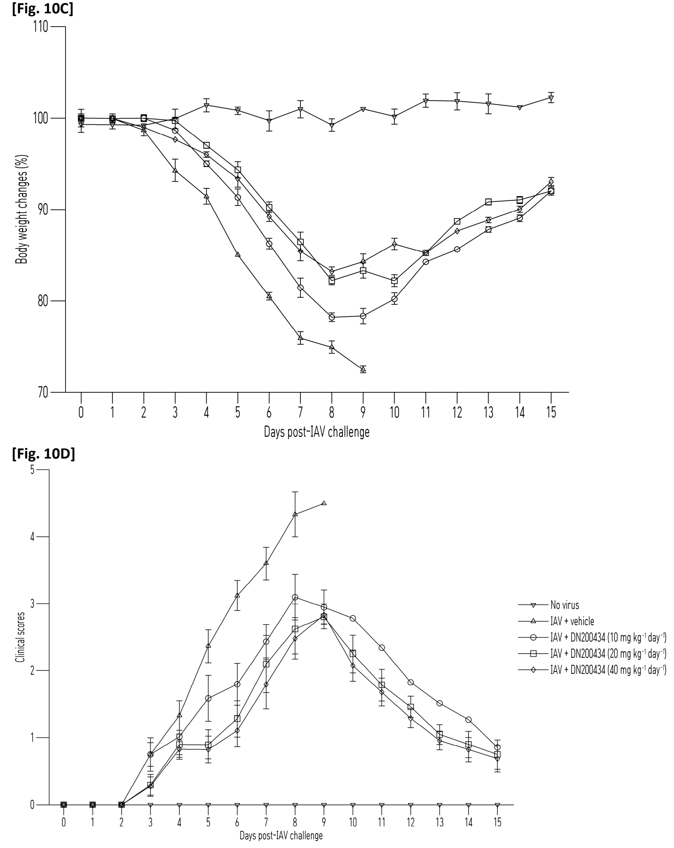

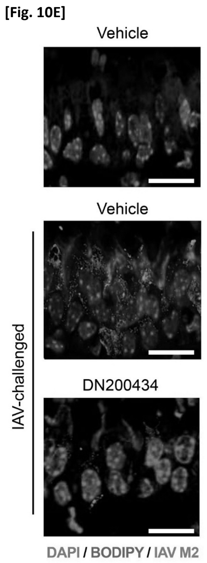

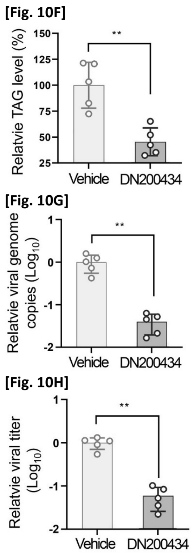

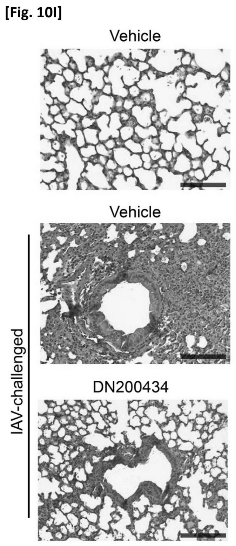

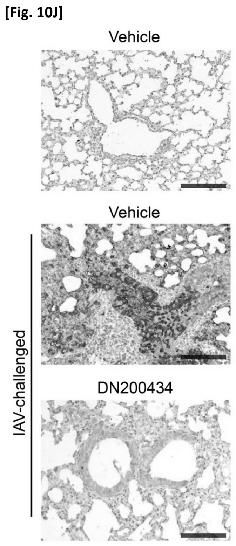

**FIG. 10A to FIG. 10J** are experimental results showing that in vivo antiviral effects of DN200434 against influenza A virus (IAV), in an example of the present disclosure. (FIG. 10A) Scheme of chemical administration twice daily for four consecutive days after challenge with 10³ PFU of mouse-adapted IAV PR8 strain to mice (n = 16). **(****FIGS. 10B-10D****)** Comparison of survival rates (FIG. 10B), body weight changes (FIG. 10C), and clinical scores (FIG. 10D) between mock-treated and DN200434-treated, IAV-challenged mice. **(****FIG. 10E****)** Representative confocal images of dose-dependent reduction in BODIPY-stained LDs (green) and IAV replication (red) in bronchiolar epithelial cells of lung tissues sampled from IAV-challenged mice by treatment with DN200434. **(****FIGS. 10F-10H****)** Graphical representation of dose-dependent reduction in TAG (FIG. 10F), viral genome copy numbers (FIG. 10G), and infectious progeny titers (FIG. 10H) in lungs sampled from IAV-challenged mice (*n* = 5) by treatment with DN200434. **(****FIGS. 10I****, 10J)** Representative images of histological lung lesions (FIG. 10I) and immunohistochemical antigen distribution of IAV (FIG. 10J). Amelioration of histological lung lesions and inhibition of IAV replication by treatment with DN200434.

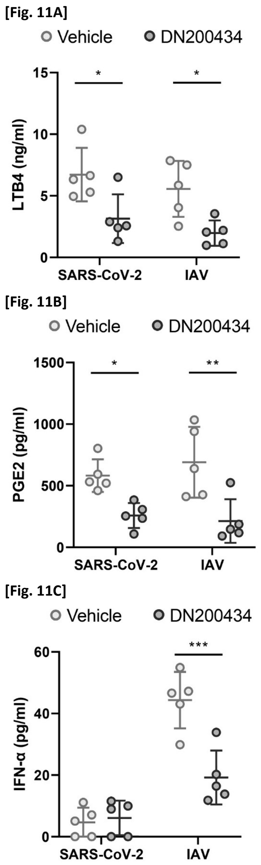

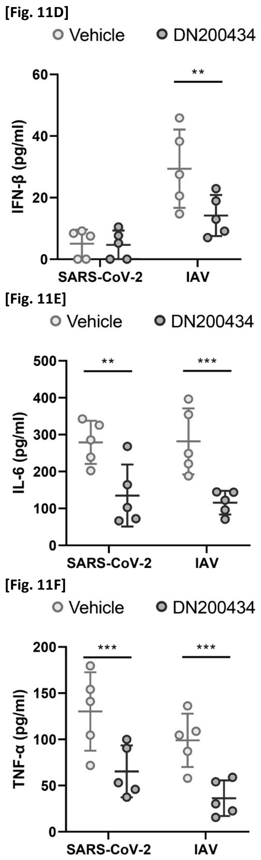

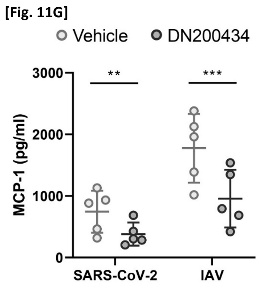

**FIG. 11A** to **FIG. 11G** presents experimental results showing that, in vivo, DN200434 has anti-eicosanoid and anti-inflammatory cytokine effects against SARS-CoV-2 and influenza A virus (IAV) infection, as an example of the present disclosure. **(****FIGS. 11A-11G****)** The graphical representation of the LTB4 (FIG. 11A), PGE2 (FIG. 11B), IFN-α (FIG. 11C), IFN-β (FIG. 11D), IL-6 (FIG. 11E), TNF-α (FIG. 11F), and MCP-1 (FIG. 11G) levels in bronchoalveolar lavage fluids (BALFs) sampled from either SARS-CoV-2-challenged hamsters or IAV-challenged mice, which were vehicle-treated or treated with DN200434.

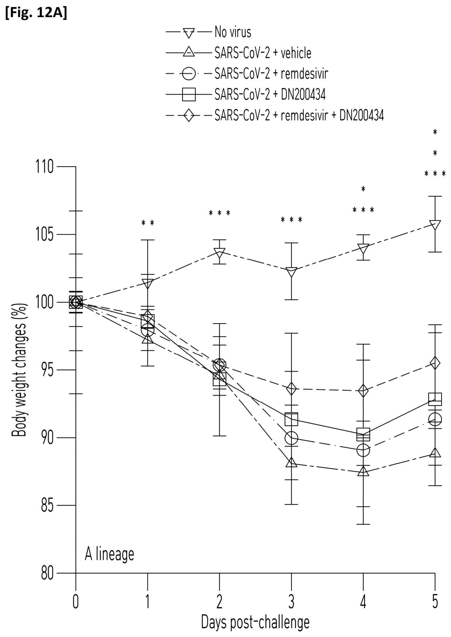

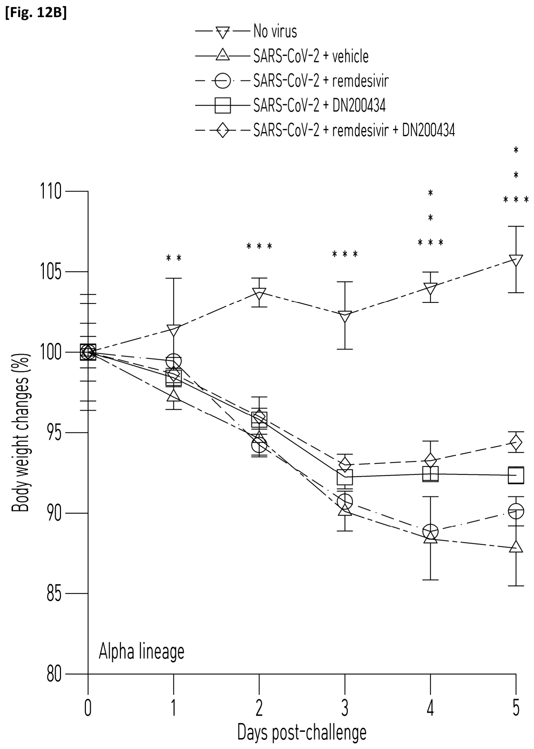

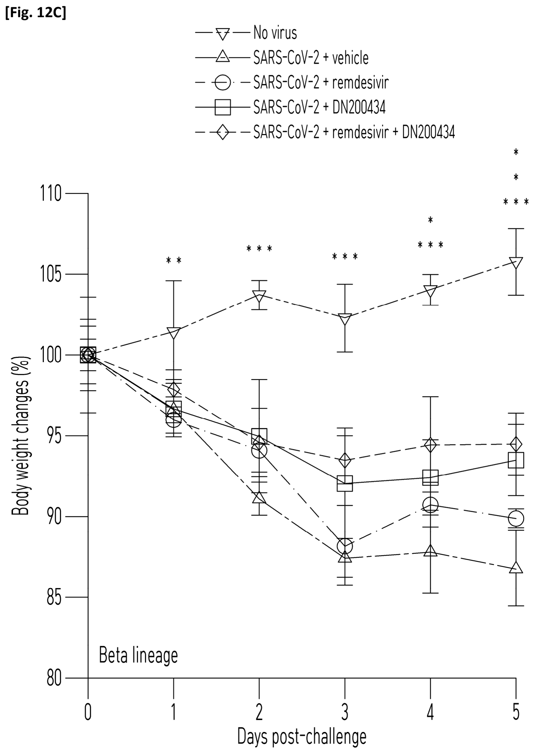

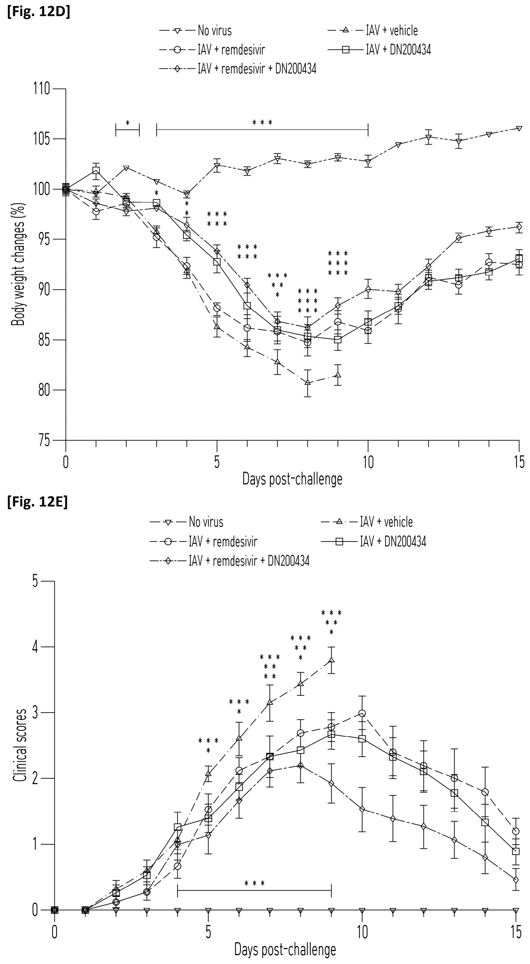

**FIG. 12A** to **FIG. 12E** presents experimental results showing improvements in body weight loss and clinical signs with treatment with DN200434 in SARS-CoV-2-challenged hamsters and IAV-challenged mice, as an example of the present disclosure. **(****FIGS. 12A-12C****)** Effect of treatments on the recovery of body weight in SARS-CoV-2 KCDC03 strain-challenged (FIG. 12A), KDCA51463 strain-challenged (FIG. 12B), and KDCA55905 strain-challenged (FIG. 12C) groups. **(****FIGS. 12D****,** **12E)** Effect of treatments on the recovery of body weight (FIG. 12D) and the reduction in clinical signs (FIG. 12E) in each group.

### BEST MODE FOR CARRYING OUT THE INVENTION

Hereinafter, embodiments and examples of the present disclosure will be described in detail with reference to the accompanying drawings so that the present disclosure may be readily implemented by those skilled in the art. However, it is to be noted that the present disclosure is not limited to the embodiments and examples but can be embodied in various other ways. In drawings, parts irrelevant to the description are omitted for the simplicity of explanation, and like reference numerals denote like parts throughout the whole document.

Throughout the whole document, the term "comprises or includes" and/or "comprising or including" used in the document means that one or more other components, steps, operation and/or existence or addition of elements are not excluded in addition to the described components, steps, operation and/or elements unless context dictates otherwise.

Throughout the whole document, the term "about or approximately" or "substantially" is intended to have meanings close to numerical values or ranges specified with an allowable error and intended to prevent accurate or absolute numerical values disclosed for understanding of the present disclosure from being illegally or unfairly used by any unconscionable third party.

Throughout the whole document, the term "step of" does not mean "step for".

Through the whole document, the term "combination(s) of" included in Markush type description means mixture or combination of one or more components, steps, operations and/or elements selected from a group consisting of components, steps, operation and/or elements described in Markush type and thereby means that the disclosure includes one or more components, steps, operations and/or elements selected from the Markush group.

Throughout the whole document, a phrase in the form "A and/or B" means "A or B, or A and B".

Throughout the whole document, the term "alkyl" or "alkyl group" may individually include linear or branched alkyl groups having 1 to 12 carbon atoms, 1 to 10 carbon atoms, 1 to 8 carbon atoms, or 1 to 5 carbon atoms, and all the possible isomers thereof. For example, the alkyl or alkyl group may individually include methyl group (Me), ethyl group (Et), n-propyl group (ⁿPr), iso-propyl group (ⁱPr), n-butyl group (ⁿBu), iso-butyl group (ⁱBu), tert-butyl group (^{t}Bu), sec-butyl group (^{s}Bu), n-pentyl group (ⁿPe), iso-pentyl group (ⁱPe), sec-pentyl group (^{s}Pe), tert-pentyl group (^{t}Pe), n-hexyl group, iso-hexyl group, heptyl group, 4,4-dimethyl pentyl group, octyl group, 2,2,4-trimethyl pentyl group, nonyl group, decyl group, undecyl group, dodecyl group, and isomers thereof, but may not be limited thereto.

Throughout the whole document, the symbol "%" used to indicate the concentration of a particular substance, unless otherwise stated, refers to (wt/wt) % for solid/solid, (wt/vol) % for solid/liquid, and (vol/vol) % for liquid/liquid throughout the present specification.

Throughout the whole document, to increase the reliability of the results under identical conditions, three independent experiments were conducted. The values for each group were converted to mean ± standard deviation. The statistical significance of the above results was analyzed using the One-Way ANOVA test (GraphPad Prism software, version 8.4.2), and p-values of 0.05 or higher were considered statistically significant.

Hereinafter, embodiments of the present disclosure are described in detail. However, the present disclosure is not limited thereto.

An aspect of the present disclosure provides an antiviral pharmaceutical composition including an estrogen-related receptor γ (ERRy) inhibitor as an active ingredient.

The present inventors discovered that, in virus-infected cells, particularly RNA virus-infected cells, ERRy increases from the early to the middle stages of infection and migrates into the nucleus. As a result, ERRy was found to induce transcription of sterol regulatory element-binding protein 1c (SREBP1c), thereby activating fatty acid (FA) biosynthesis and reprogramming host cell physiology, both of which are essential for viral replication.

From this discovery, the present inventors confirmed that inhibition of ERRy exhibits broad-spectrum antiviral effects, and thus conceived an antiviral pharmaceutical composition containing, as an active ingredient, an ERRy (estrogen-related receptor y) inhibitor of the present disclosure. Accordingly, throughout the whole document, the term "ERRy inhibitor" may collectively refer to agents that reduce ERRy expression at the transcriptional level or interfere with ERRy activity, thereby decreasing ERRy expression or activity.

Throughout the whole document, the term "broad-spectrum antiviral effects" refers to the ability to inhibit the proliferation of viruses, particularly all RNA viruses, regardless of the presence or absence of an outer membrane, whether the viral genome is positive-sense, negative-sense, or double-stranded, and whether it is single-stranded linear or segmented.

In an embodiment of the present disclosure, the ERRy inhibitor may inhibit the activity of the ERRy protein or suppress the expression of the ERRy gene.

In an embodiment of the present disclosure, the ERRy inhibitor may exert antiviral effects by inhibiting ERRy activity in virus-infected cells.

In an embodiment of the present disclosure, the ERRy inhibitor may include an siRNA specific to ERRy.

In an embodiment of the present disclosure, the siRNA may be a nucleic acid molecule capable of mediating RNA interference or gene silencing. Because it can suppress the expression of a target gene, it can be used as an efficient gene knockdown method or as a gene therapy approach.

In an embodiment of the present disclosure, the ERRy inhibitor may include an inverse agonist of ERRy.

In an embodiment of the present disclosure, the ERRy inverse agonist may include a compound represented by the following Chemical Formula 1:

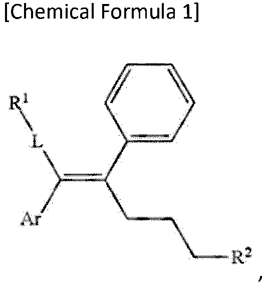

In Chemical Formula 1,
L is (C₆-C₂₀)arylene, (C₃-C₂₀)heteroarylene, or (C₃-C₂₀) fused heterocycle;
R¹ is (C₃-C₂₀)heterocycloalkyl, (C₃-C₂₀)heteroaryl, -O-(CH₂)ₘ-R¹¹, -(CH₂)ₘ-R¹², -NH-(CH₂)ₘ-R¹³, -NHCO-(CH₂)ₙ-R¹⁴, or -SiR¹⁶R¹⁷-(CH₂)ₘ-R¹⁵;
R¹¹to R¹⁵ are each, independently, (C₃-C₂₀)heterocycloalkyl;
R¹⁶ and R¹⁷ are each, independently, (C₁-C₂₀)alkyl;
m is an integer of 1 to 3; and
n is an integer of 0 or 1;
Ar is (C₆-C₂₀)aryl or (C₃-C₂₀)heteroaryl,
in which the aryl or heteroaryl of Ar may be unsubstituted or substituted with one or more substituents selected from hydroxy, halogen, (C₁-C₂₀)alkyl, halo(C₁-C₂₀)alkyl, (C₁-C₂₀)alkoxy, nitro, cyano, -NR²¹R²², (C₁-C₂₀)alkylcarbonyloxy, (C₁-C₂₀)alkylcarbonylamino, guanidino, - SO₂-R²³, and -OSO₂-R²⁴,
R²¹ and R²² are each, independently, hydrogen, (C₁-C₂₀)alkylsulfonyl, or (C₃-C₂₀)cycloalkylsulfonyl;
R²³ and R²⁴ are each, independently, (C₁-C₂₀)alkyl, halo(C₁-C₂₀)alkyl, or (C₃-C₂₀)cycloalkyl;
R²is hydroxy, halogen, (C₁-C₂₀)alkylcarbonyloxy, or (C₁-C₂₀)alkylsulfonyloxy;
the heterocycloalkyl or heteroaryl of R¹ and the heterocycloalkyl of R¹¹to R¹⁵ may be unsubstituted or substituted with one or more substituents selected from (C₁-C₂₀)alkyl, (C₃-C₂₀)cycloalkyl, (C₂-C₂₀)alkenyl, amidino, (C₁-C₂₀)alkoxycarbonyl, hydroxy, hydroxy(C₁-C₂₀)alkyl, and di(C₁-C₂₀)alkylamino(C₁-C₂₀)alkyl, and
the heterocycloalkyl and heteroaryl contain one or more heteroatoms selected from N, O and S, and
the heterocycloalkyl is a saturated or unsaturated mono-, bi-, or spirocycle having a carbon atom or nitrogen atom in a ring as a binding site.

In an embodiment of the present disclosure, the ERRy inverse agonist may include a compound represented by the following Chemical Formula 2:

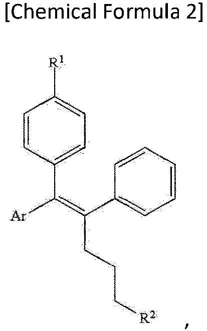

In Chemical Formula 2,
R¹ is (C₃-C₂₀)heterocycloalkyl, (C₃-C₂₀)heteroaryl, -O-(CH₂)ₘ-R¹¹, -(CH₂)ₘ-R¹², -NH-(CH₂)ₘ-R¹³, -NHCO-(CH₂)ₙ-R¹⁴, or -SiR¹⁶R¹⁷-(CH₂)ₘ-R¹⁵;
R¹¹ to R¹⁵ are each, independently, (C₃-C₂₀)heterocycloalkyl;
R¹⁶ and R¹⁷ are each, independently, (C₁-C₂₀)alkyl;
m is an integer of 1 to 3; and
n is an integer of 0 or 1;
Ar is (C₆-C₂₀)aryl or (C₃-C₂₀)heteroaryl,
in which the aryl or heteroaryl of Ar may be unsubstituted or substituted with one or more substituents selected from hydroxy, halogen, (C₁-C₂₀)alkyl, halo(C₁-C₂₀)alkyl, (C₁-C₂₀)alkoxy, nitro, cyano, -NR²¹R²², (C₁-C₂₀)alkylcarbonyloxy, (C₁-C₂₀)alkylcarbonylamino, guanidino, - SO₂-R²³, and -OSO₂-R²⁴,
R²¹ and R²² are each, independently, hydrogen, (C₁-C₂₀)alkylsulfonyl, or (C₃-C₂₀)cycloalkylsulfonyl;
R²³ and R²⁴ are each, independently, (C₁-C₂₀)alkyl, halo(C₁-C₂₀)alkyl, or (C₃-C₂₀)cycloalkyl;
R² is hydroxy, halogen, (C₁-C₂₀)alkylcarbonyloxy, or (C₁-C₂₀)alkylsulfonyloxy;
the heterocycloalkyl or heteroaryl of R¹ and the heterocycloalkyl of R¹¹ to R¹⁵ may be substituted or not substituted with one or more substituents selected from (C₁-C₂₀)alkyl, (C₃-C₂₀)cycloalkyl, (C₂-C₂₀)alkenyl, amidino, (C₁-C₂₀)alkoxycarbonyl, hydroxy, hydroxy(C₁-C₂₀)alkyl, and di(C₁-C₂₀)alkylamino(C₁-C₂₀)alkyl, and
the heterocycloalkyl and heteroaryl contain one or more heteroatoms selected from N, O and S, and
the heterocycloalkyl is a saturated or unsaturated mono-, bi-, or spirocycle having a carbon atom or nitrogen atom in a ring as a binding site.

In an embodiment of the present disclosure, the ERRy inverse agonist may include DN200434 and/or GSK5182, but is not limited thereto.

In an embodiment of the present disclosure, the DN200434 may be represented by the following Chemical Formula 3:

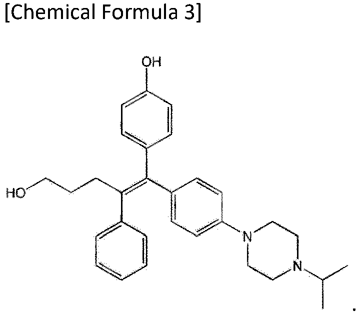

In an embodiment of the present disclosure, the GSK5182 may be represented by the following Chemical Formula 4:

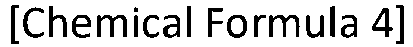

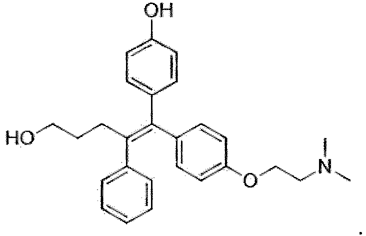

In an embodiment of the present disclosure, the pharmaceutical composition may be for the prevention or treatment of RNA virus infections.

In an embodiment of the present disclosure, the RNA virus may be at least one selected from SARS-CoV-1, SARS-CoV-2, influenza A virus (IAV), influenza B virus, bovine coronavirus (BCoV), porcine epidemic diarrhea coronavirus (PEDV), bovine species A rotavirus (RVA), porcine reproductive and respiratory syndrome virus (PRRSV), porcine sapovirus (PSaV), norovirus (NoV), feline infectious peritonitis virus (FIPV), MERS-related coronavirus (MERS-CoV), Zika virus (ZIKV), dengue virus, human torovirus (HuTV), hepatitis A virus (HAV), and hepatitis C virus (HCV).

Herein, the term "coronavirus" refers to viruses within the subfamily Orthocoronavirinae of the family Coronaviridae. These viruses are classified into four genera: alpha-, beta-, gamma-, and delta-coronavirus. Within the alpha-coronavirus genus, there are viruses such as PEDV and FIPV. BCoV, SARS-CoV-1, SARS-CoV-2, and MERS-CoV fall within the beta-coronavirus genus. Representatives of the gamma-coronavirus genus include the chicken infectious bronchitis virus (IBV), and examples of the delta-coronavirus genus include human and pig delta-coronaviruses. Coronaviruses have a size range of 80 nm to 120 nm and possess spikes on their envelope. Bovine coronaviruses, which belong to the gamma-coronavirus genus, additionally feature an external hemagglutinin/esterase protein spike. Inside the envelope, there is a positive-sense, single-stranded RNA genome.

Herein, the term "influenza A virus" refers to a virus of the Alphainfluenzavirus genus within the Orthomyxoviridae family. This virus is 80-120 nm in size. The surface of the virus's envelope has hemagglutinin and neuraminidase spikes oriented outwardly. Inside the envelope, there are eight segments of a negative-sense genome. The influenza A virus infects various mammals, including humans, pigs, horses, and dogs, as well as birds such as chickens.

Herein, the term "influenza B virus" refers to a virus of the Betainfluenzavirus genus within the Orthomyxoviridae family. This virus is 80-120 nm in size. The surface of the virus's envelope has hemagglutinin and neuraminidase spikes oriented outwardly. Inside the envelope, there are eight segments of a negative-sense genome. Unlike the influenza A virus, the influenza B virus infects only a limited range of species, such as humans, martens, pigs, and seals.

Herein, the term "BCoV" denotes a virus that causes severe diarrhea in calves and mature cows worldwide.

Herein, the term "PEDV" denotes a virus that causes diarrhea across all age groups worldwide and leads to a mortality rate of 60% or more in suckling pigs.

Herein, the term "rotavirus" refers typically to species A rotavirus (RVA). Within the Rotavirus genus in the Reoviridae family, there are nine species: A, B, C, D, F, G, H, I, and J. Among them, humans are primarily infected by species A rotavirus. Species A through I rotaviruses cause diseases in animals other than humans. It has been reported that the H type infects pigs, the D, F, and G types infect birds, the I type infects cats, and the J type infects bats. Rotavirus possesses 11 segments of a double-stranded genome inside three 80 nm capsid layers. Rotavirus is a major cause of diarrhea in infants under 5 years old and in young livestock. For example, the rotavirus may include bovine species A rotavirus (RVA).

Herein, the term "PRRSV" belongs to the Arterivirus genus of the Arteriviridae family. It is classified into Type 1 (European type) and Type 2 (North American type). PRRSV has an envelope size of 50-60 nm, with 4 envelope protein and glycoprotein spikes. Inside the envelope is a positive-sense, single-stranded RNA genome approximately 15 kb in length. PRRSV induces reproductive disorders, such as abortion and stillbirth, in pregnant sows and interstitial pneumonia in piglets.

Herein, the term "sapovirus" refers to a virus belonging to the Sapovirus genus within the Caliciviridae family. It is a causative agent of diarrhea in humans, pigs, and minks.

Herein, the term "FIPV" denotes a viral causative agent that causes vasculitis in cats, leading to severe pleural and abdominal effusions or chronic granulomatous nodules in various organs such as the kidneys.

Herein, the term "caliciviruses" refers to viruses within the Caliciviridae family, which possess a linear, positive-sense, single-stranded RNA genome within a capsid layer of 27 nm to 40 nm in size. Within the Caliciviridae family, there are currently 11 recognized genera: *Norovirus, Sapovirus, Vesivirus, Lagovirus, Nebovirus, Recovirus, Valovirus, Babovirus, Nacovirus, Solovirus,* and *Minovirus.*

Herein, the term "torovirus" refers to viruses within the subfamily *Torovirinae* of the family *Tobaniviridae.* The subfamily *Torovirinae* includes bovine torovirus (BToV), equine torovirus (EToV), porcine torovirus (PToV), and human torovirus (HuTV). Toroviruses are 100 nm to 150 nm in size and possess spikes and hemagglutinin/esterase protein spikes on their envelope. Inside the envelope, there is a positive-sense, single-stranded RNA genome.

In an embodiment of the present disclosure, the pharmaceutical composition may further include an antiviral agent.

In an embodiment of the present disclosure, the antiviral agent may include one or more selected from oseltamivir and remdesivir.

In an embodiment of the present disclosure, wherein the antiviral agent is oseltamivir, the volume ratio of the ERRy inverse agonist to oseltamivir may be about 2:2 to about 8:2, but is not limited thereto.

In an embodiment of the present disclosure, wherein the antiviral agent is oseltamivir, the volume ratio of the ERRy inverse agonist to oseltamivir may be about 2:2 to about 8:2, preferably about 3:2 to about 7:2, or more preferably about 4:2 to about 6:2, but is not limited thereto.

In an embodiment of the present disclosure, wherein the antiviral agent is remdesivir, the volume ratio of the ERRy inverse agonist to remdesivir may be about 8:1 to about 20:1, but is not limited thereto.

In an embodiment of the present disclosure, wherein the antiviral agent is remdesivir, the volume ratio of the ERRy inverse agonist to remdesivir may be about 8:1 to about 20:1, preferably about 10:1 to about 18:1, or more preferably about 15:1 to about 16:1, but is not limited thereto.

Hereinafter, the present disclosure will be described in more detail with reference to Examples, but the present disclosure may not be limited thereto.

### MODE FOR CARRYING OUT THE INVENTION

### [Examples]

### 1. Materials and Methods

### Cells and Viruses

Vero E6, MDCK, LLC-PK, MA104, A549, Caco-2, MARC-145, and HRT-18G cells were cultured in EMEM, α-MEM, or DMEM at 37°C with 5% CO₂. All media were supplemented with 10% fetal bovine serum, 100 U/mL penicillin, and 100 µg/mL streptomycin. In the present disclosure, the following strains were used: SARS-CoV-2 (KCDC03, KDCA51463, and KDCA55905 strains), IAV [PR8/34 (H1N1) strain], BCoV (KWD20 strain), PEDV (QIAP1401 strain), PRRSV (LYM strain), RVA (NCDV strain), and PSaV (Cowden strain).

### 2. Results

### (1) Dynamics of ERRy expression are a common occurrence in RNA virus infections

While certain NRs have been reported to exhibit proviral, antiviral, or dual activities depending on the virus types and their target host, the role of ERRs in viral infections remains unexplored. Before investigating the association between ERRs and viral replication, we evaluated the sequential expression levels of ERRs during the replication of seven target RNA viruses. In cells infected with SARS-CoV-2 and IAV, ERRy protein expression exhibited distinct dynamics: gradually increasing and then declining during viral replication (**FIGS. 1A****,** **1B**). Notably, ERRy, increased in response to viral replication, underwent translocation into nuclear (**FIGS. 1A-1D**), suggesting that it might play a key role in virus infection. Other target RNA viruses, including influenza A virus, bovine coronavirus (BCoV), porcine epidemic diarrhea coronavirus (PEDV), bovine species A rotavirus (RVA), porcine reproductive and respiratory syndrome virus (PRRSV), and porcine sapovirus (PSaV), showed a similar ERRy expression pattern in infected cells.

To confirm whether ERRy dynamics were also evident in virus-infected animals, we examined alveolar pneumocytes in SARS-CoV-2-challenged hamsters and bronchiolar epithelial cells in IAV-challenged mice. There was a gradual increase in ERRy expression during the early phase of infection, followed by a decline in the late phase (**FIGS. 1E****,** **1F**). Importantly, the translocation of ERRy from the cytoplasm to the nucleus was enhanced in infected animals (**FIGS. 1E-1H**). In parallel, *ERRγ* mRNA levels in the lungs of SARS-CoV-2 and IAV-infected animals showed trends similar to the *ERRγ* protein levels observed in SARS-CoV-2 and IAV-infected cells. Intriguingly, *ERRα* mRNA levels remained unaffected during viral replication. These results suggest that, notably, the biphasic expression pattern of ERRy is a common feature of RNA virus infections.

### (2) Upregulation of ERRy through activation of ROS-induced JNK/c-Jun signaling pathway

Next, we aimed to uncover the pathway through which viral infections upregulate ERRy expression. Oxidative stress induced by ROS is a common pathophysiological feature in various viral infections. Notably, ERRy has been reported to function as a ROS sensor, implying that virus-induced ROS induction could potentially upregulate ERRy. In the present disclosure, IAV-infected cells showed an increase in ROS levels to a maximum at 8 hpi, remaining high until 12 hpi (**FIG. 2A**). SARS-CoV-2-infected cells exhibited a steady increase in ROS levels up to 12 hpi (**FIG. 2A**). Furthermore, treatment with the antioxidant N-acetylcysteine (NAC) significantly suppressed virus-induced ROS generation (**FIG. 2B**).

To determine if virus-induced ROS indeed transactivates ERRy, cells were left mock-transfected or transfected with a plasmid containing the full-length *ERRγ* promoter-luciferase gene (*ERRγ*-luc). The cells in the above conditions were infected with either IAV or SARS-CoV-2, with or without NAC. Both viruses significantly activated the *ERRγ* promoter and enhanced ERRy expression, and in both cases, activation was attenuated by NAC treatment (**FIGS. 2C****,** **2D**). Since the *ERRγ* promoter is known to be activated by the JNK/c-Jun signaling pathway, we examined whether virus-induced ERRy activation occurs through this pathway. Interestingly, infection with either IAV or SARS-CoV-2 induced phosphorylation of both JNK (p-JNK) and c-Jun (p-c-Jun) at 4 hpi and 8 hpi, respectively (**FIG. 2E**). Additionally, a mutation in the c-Jun binding element AP1 on the *ERRγ* promoter (*ERRγ-*AP1mut-luc) reduced *ERRγ*-luc activity in cells infected with either SARS-CoV-2 or IAV (**FIG. 2F**). Chromatin immunoprecipitation (ChIP) assays demonstrated that both viruses increased the occupancy of c-Jun on the AP1 regulatory element site of the *ERRγ* promoter and that this effect was markedly blocked by NAC treatment (**FIG. 2G**). These data suggest that virus-induced ERRy transactivation primarily occurs through the ROS/JNK/c-Jun signaling pathway, culminating in c-Jun directly activating ERRy expression.

### (3) ERRy deficiency renders the mice resistant to IAV replication in vitro and in vivo

To directly investigate the role of ERRy in viral replication, we first knocked down ERRy *in vitro* and infected cells with target viruses. Knockdown of ERRy had a profound inhibitory effect on the expression levels of viral proteins and the viral genome copy number (**FIGS. 3A-3C**). Subsequently, we employed a loss-of-function approach to determine whether ERRy knockout (KO) mice exhibit resistance to IAV infection. We utilized heterozygous mice *(Esrrg*^{*+*/}*⁻)* because homozygous ERRy-null mice die shortly after birth. These *Esrrg*^{*+*/*-*} mice exhibited reduced ERRy expression in their lungs compared with wild-type (WT) controls. Given the unavailability of a double transgenic mouse simultaneously heterozygous for ERRy and human ACE2, which would be required for the SARS-CoV-2 challenge study, we selected the IAV PR8/34 (H1N1) strain as a representative RNA virus that infects WT mice. The intranasal challenge of WT mice with 10³ PFU of IAV PR8 strain led to 100% mortality within 10 days after the challenge, accompanied by severe body weight loss and clinical signs (**FIGS. 3D-3F**). In contrast, *Esrrg*^{*+*/*-*} mice showed a marked reduction in mortality (50%), maintained body weight, and exhibited less severe clinical signs (**FIGS. 3D-3F**). Furthermore, compared with WT mice, ERRy deficiency resulted in diminished IAV protein synthesis, viral genome replication, and progeny viral production in the lungs (**FIGS. 3G-3I**). Histopathological lung lesions induced by IAV infection in the WT mice were significantly ameliorated in ERRy-deficient mice (**FIG. 3J**), resulting from a decrease of IAV antigen-positive cells (**FIG. 3K**). These data strongly suggest that ERRy promotes the replication of RNA viruses.

### (4) Virus-induced ERRγ transactivates SREBP1c, leading to FA biosynthesis required for viral replication

In our previous studies, we established that ERRy is a transcriptional regulator of SREBP1c, a crucial transcription factor that activates FA biosynthesis, and that its misregulation contributes to several fatty liver conditions. As noted in previous reports, we have confirmed that infection with RNA viruses can stimulate lipid droplet (LD) formation via lipogenesis, both in vitro and in vivo. To investigate whether ERRy regulates viral infection by modulating SREBP1c, we initially examined SREBP1c dynamics in virus-infected cells. Similar to ERRy dynamics, we observed an initial increase followed by a subsequent decline at a later stage in both SREBP1 protein and SREBP1c transcriptional levels in cells infected with the seven target RNA viruses, including SARS-CoV-2 or IAV (**FIGS. 4A-4C**)**.** Notably, the increased SREBP1 subsequently translocated into the nucleus in RNA virus-infected cells. This pattern was also evident in lung samples from mice challenged with IAV (**FIGS. 4D-****4F**) and hamsters challenged with SARS-CoV-2. However, activation of the SREBP1a isoform was not observed in vitro and in vivo.

Next, we sought to confirm whether ERRy directly regulates the SREBP1c gene in virus-infected cells. We transfected cells with a luciferase reporter vector for the human SREBP1c gene promoter (SREBP1c-luc) and then infected the cells with each of the target RNA viruses. SREBP1c promoter activity was significantly enhanced by the infection with all seven target viruses, similar to the positive control (Flag-ERRy) of ERRy overexpression (**FIGS. 4G****,** **9A**). In contrast, mutating the ERR-response element (ERRE) from the SREBP1c gene (SREBP1c-ERREmut-luc) led to a significant reduction in activity (**FIGS. 4G****,** **9A**). Also, treatment with the ERRy inverse agonist DN200434 markedly suppressed the activity of the SREBP1c gene promoter in the cells infected with each individual virus (**FIGS. 4H****,** **9B**)**.** In addition, both DN200434 and ERRy siRNA reduced SREBP1c expression activated by individual virus infections (**FIGS. 4I****,** **9C**). These findings suggest that ERRy directly transactivates the SREBP1c gene in response to RNA viral infections.

In addition to ERRy-mediated transactivation of SREBP1c, we also observed that viral infections significantly increased the mRNA expression of lipogenic genes, including FASN (encoding fatty acid synthase), DGAT1 (encoding diacylglycerol O-acyltransferase 1), and SCD1 (encoding stearoyl-CoA desaturase 1) (**FIGS. 9D-9F**), as well as leading to increased intracellular triacylglycerol (TAG) levels (**FIG. 4J**). Treatment with DN200434 effectively attenuated both lipogenic gene activation and lipid accumulation in virus-infected cells (**FIG. 4J****,** **FIGS. 9C-9G**). However, the basal level of SREBP1a mRNA in SARS-CoV-2- or IAV-infected cells was not affected by treatment with DN200434 (**FIG. 9H**). Moreover, we examined whether the resistance to IAV infection observed in Esrrg+/- mice could be attributed to the failure of the ERRy-activated FA biosynthesis. As a result, ERRy knockout inhibited SREBP1c activation and decreased TAG levels and intracellular LD formation (**FIGS. 9I, 9J**).

To investigate the role of SREBP1c in viral replication in vivo, we utilized Srebp1c KO mice. Srebp1c knockout was confirmed by PCR genotyping and immunoblotting in the lung samples of Srebp1c KO mice. Notably, 100% mortality observed in IAV-challenged WT mice was reduced to 25% in the Srebp1c KO mice. In addition, IAV-challenged Srebp1c KO mice exhibited significant improvements in body weight loss and clinical signs 4 days post-challenge compared with WT IAV-infected mice. In the absence of the SREBP1c protein, viral protein synthesis, genome replication, and progeny production all significantly decreased, which was accompanied by a drastic reduction in LD formation and TAG production. Concurrently, IAV-infected Srebp1c KO mice displayed reduced histopathological lung lesions, such as bronchopneumonia and interstitial inflammatory infiltration, along with substantially diminished viral replication in the bronchioles and alveoli, compared to WT IAV-infected mice. Taken together, these findings highlight that the sequential pathway beginning with ROS activation of ERRy, which activates SREBP1c, followed by increased FA biosynthesis and subsequent LD accumulation, is essential for RNA viral replication.

### (5) In vitro broad-spectrum antiviral activity of DN200434

The studies so far suggest that inhibiting ERRy limits the replication of various RNA viruses. Thus, we assessed the antiviral effect of the ERRy inverse agonist DN200434 on in vitro replication of seven target RNA viruses, including SARS-CoV-2, IAV, BCoV, PEDV, RVA, PRRSV, and PSaV. To evaluate its performance, we measured key parameters, including the half-maximal cytotoxic concentration (CC50), the half-maximal inhibitory concentration (IC50), and the selectivity index (SI). Remarkably, DN200434 showed IC₅₀ values against the target RNA viruses at substantially lower micromolar concentrations than CC₅₀ (**FIGS. 5A-5I**). Specifically, the IC₅₀ values ranged from 1.86 ± 0.80 µM for SARS-CoV-2 to 6.24 ± 1.07 µM for PSaV. These results yielded significant SI values of 50 against SARS-CoV-2 and 34 against IAV, suggesting the broad-spectrum antiviral potential of DN200434 (**FIGS. 5A-5I**).

We investigated whether DN200434 inhibits the SARS-CoV-2 prototype and its variants. In a dose-dependent manner, DN200434 effectively suppressed genome replication, progeny production, and protein synthesis of the SARS-CoV-2 prototype strain in the A lineage, as well as two variants in the alpha and beta lineages (**FIGS. 6A-6C**). At a maximum dose of 20 µM, it resulted in a remarkable reduction in viral genome copy numbers and titers of BCoV in the genus betacoronavirus and PEDV in the genus alphacoronavirus by more than 2.0-log10 steps (**FIGS. 5H****,** **5I**). These findings indicate that DN200434 has broad-spectrum antiviral effects against coronaviruses.

Furthermore, we explored DN200434's efficacy against IAV, which poses an ongoing threat to human health and has the potential to cause lethal pandemics. Interestingly, treatment with DN200434 resulted in a dose-dependent suppression of viral genome replication, progeny production, and protein synthesis in multiple IAV-infected cell lines, including human lung epithelial (A549) and intestinal (Caco-2) cells, canine kidney epithelial (MDCK) cells, and monkey kidney epithelial (Vero E6) cells (**FIGS. 6D-6F**), demonstrating DN200434's anti-influenza virus efficacy against IAV in both human and animal cells. Taken together, our data provide strong evidence for the in vitro broad-spectrum antiviral potential of DN200434, most notably targeting SARS-CoV-2 and IAV.

### (6) Role of DN200434 in modulating viral replication and pathogenicity in vitro

Positive-sense RNA viruses rely on cellular lipids to assemble intracellular membrane structures called viral factories. These structures serve as anchoring scaffolds for viral replication and transcription complexes, such as double-membrane vesicles (DMVs) induced by coronaviruses. Additionally, FAs play a pivotal role in the palmitoylation of viral proteins, including the SARS-CoV-2 spike (S) protein, the IAV hemagglutinin (HA) protein, and the ion-channel M2 protein, facilitating crucial processes such as viral membrane fusion, assembly, budding, and virulence. In contrast, disrupting FA biosynthesis can impede DMV formation in SARS-CoV-2-infected cells and the palmitoylation of SARS-CoV-2 S protein and IAV HA and M2 proteins. We observed that DN200434 treatment led to a significant reduction in SARS-CoV-2-induced perinuclear DMV clusters containing viral double-stranded RNA, as assessed by transmission electron microscopy (TEM) and confocal microscopy (**FIGS. 6G****,** **6H**). Furthermore, TEM analysis demonstrated a notable reduction in the number of progeny viral particles in DN200434-treated cells compared with vehicle-treated virus-infected cells (**lower two panels in** **FIG. 6G**). Additionally, the palmitoylation of SARS-CoV-2 S protein and IAV HA proteins, typically observed in vehicle-treated, virus-infected cells, was significantly inhibited by DN200434 (**FIGS. 6I, 6J**). Furthermore, treatment with DN200434 in SARS-CoV-2- and IAV-infected cells inhibited energy production through β-oxidation in the mitochondria from the FFA substrate (**FIG. 6K**).

We next investigated the role of DN200434 in reducing the excessive proinflammatory cytokine secretion, known as a cytokine storm, which contributes to severe infections and increased mortality in patients with SARS-CoV-2 and IAV infections. Proinflammatory cytokines can be induced directly by cytoplasmic sensors that respond to viral components, as well as indirectly through the production of eicosanoids, including FA-derived lipid mediators such as prostaglandin E2 (PGE2) and leukotriene B4 (LTB4). In the present invention, treatment with DN200434 significantly reduced the elevated levels of PGE2 and LTB4 observed in SARS-CoV-2- and IAV-infected cells (**FIGS. 6L, 6M**). In IAV-infected A549 cells, moreover, DN200434 treatment led to significant suppression of proinflammatory cytokines, including interferon-α. (IFN-α), IFN-β, tumor necrosis factor-a (TNF-α), interleukin-6 (IL-6), and monocyte chemoattractant protein-1 (MCP-1), which are typically activated in response to IAV infection (**FIGS. 6N-6R**). Due to both the inability of Vero cells to synthesize IFNs and the antagonist role of SARS-CoV-2 proteins against types I and III IFNs, treatment with DN200434 significantly reduced TNF-α, IL-6, and MCP-1 levels, while IFN-α and IFN-β levels remained unchanged (**FIGS. 6N-6R**). These results suggest that DN200434 can reduce the virus-associated severe proinflammatory cytokine response, which may seriously impact mortality in COVID-19 and IAV patients.

We investigated the impact of DN200434 treatment on the FFA profiles in virus-infected cells using a gas chromatography-flame ionization detector (GC-FID). A heatmap was generated using selected FFAs that exhibited statistically significant changes (p < 0.05). Interestingly, treatment of SARS-CoV-2- or IAV-infected cells with DN200434 either restored the level of selected FFA to that of mock control cells or decreased it below mock control levels (**FIGS. 7A****,** **7B**). Particularly, the synthesis of representative fatty acids induced by SARS-CoV-2 or IAV infection was significantly interrupted by DN200434 (**FIGS. 7C-7H**). To assess the restoration of viral growth in DN200434-treated conditions, we selected four prominent FFAs: saturated palmitic acid (PA), monounsaturated oleic acid (OA), polyunsaturated linoleic acid (LA), and polyunsaturated arachidonic acid (AA). Supplementation of these individual FFAs at least partially restored genome replication and progeny production of SARS-CoV-2 and IAV in virus-infected, FFA-deprived cells, compared to DN200434-treated controls **(****FIGS. 7I-7L****).** These findings collectively suggest that DN200434, by suppressing ERRy, interferes with viral replication compartment formation, viral morphogenesis (palmitoylation), and energy generation by disrupting the downstream SREBP1c-mediated FA biosynthetic pathway.

### (7) Antiviral activity of DN200434 against SARS-CoV-2 and IAV infection in vivo

According to our previous lead compound optimization and pharmacokinetics studies, DN200434 stands out as the most potent ERRy inverse agonist, with a functional IC50 of 0.006 µM/L, which is 12-fold higher than the original lead compound GSK5182. Importantly, DN200434 has displayed promising in vitro/in vivo toxicity profiles in the required standard discovery studies. We administered DN200434 intraperitoneally (i.p.) to mice (20 mg kg⁻¹ d⁻¹) and Syrian hamsters (40 mg kg⁻¹ d⁻¹), which allowed the compound to circulate in the bloodstream and reach the lungs. Remarkably, DN200434 did not induce any signs of toxicity for 14 days in both mice and Syrian hamsters. Furthermore, treatment with DN200434 did not result in gross or histopathological changes in vital organs, including the liver, lung, kidney, heart, and spleen, in mice and hamsters.

We proceeded to investigate whether DN200434 could ameliorate gross lung lesions in the hamsters challenged with 10⁵ TCID₅₀ of the SARS-CoV-2 KCDC03 strain belonging to the A lineage of the early Chinese strains. Intraperitoneal administration of SARS-CoV-2-challenged hamsters with different concentrations of DN200434 for four and a half consecutive days (twice a day) significantly improved gross lung lesions in a dose-dependent manner. Notably, treatment with DN200434 at 40 mg kg⁻¹ d⁻¹ led to an impressive 92% reduction in gross lung lesions compared to the virus-challenged, vehicle-treated control group (**FIGS. 8A, 8B**). Next, we evaluated the antiviral effects of DN200434 against the mouse-adapted IAV PR8 strain (H1N1) in mice. While all IAV-challenged, vehicle-treated mice in the control group died within 10 days, intraperitoneal administration of DN200434 to IAV-challenged mice at different concentrations for 4 consecutive days (twice a day) improved survival in a concentration-dependent manner, reaching 40% at 20 mg kg⁻¹ d⁻¹ (**FIGS. 10A, 10B**)**.** In addition, DN200434 administration alleviated body weight loss due to SARS-CoV-2 and IAV infections, as well as clinical signs associated with IAV infection (**FIG. 8C****,** **FIGS. 10A****,** **10D**).

To investigate whether the improvements in SARS-CoV-2-induced lung lesions and IAV-induced mortality induced by DN200434 administration were linked to inhibition of viral replication, lipogenesis, and LD formation, we examined cytoplasmic lipid droplets and viral antigens in alveolar and bronchial epithelial cells in lung tissues. The results showed that DN200434 significantly reduced both LD and viral antigens in these cells (**FIGS. 8D****,** **10E**). Furthermore, DN200434 administration led to a decrease in TAG levels, a major component of LDs, in lung tissues compared to virus-challenged, vehicle-treated control animals (**FIGS. 8E****,** **10F**). Also, DN200434-induced inhibition of the intracellular LD generation in virus-challenged animals resulted in a substantial reduction in viral genome replication and progeny production (**FIGS. 8F, 8G****,** **10G, 10H**)**.** This suppression of viral replication effectively mitigated histopathological lung lesions, such as interstitial thickening, alveolar and bronchiolar epithelial cell necrosis, and lung edema (**FIGS. 8H****,** **10I****,** **10J**)**.**

Intraperitoneal administration of DN200434 to SARS-CoV-2-challenged hamsters significantly reduced the levels of LTB4, PGE2, IL-6, TNF-α, and MCP-1, while no effect was observed on IFN-α and IFN-β levels compared to virus-challenged, vehicle-treated controls (**FIGS. 11A-****11G**)**.** In IAV-challenged mice, moreover, DN200434 administration significantly suppressed the levels of LTB4, PGE2, IFN-α, IFN-β, IL-6, TNF-α, and MCP-1 compared to virus-infected, vehicle-treated controls (**FIGS. 11A-11G**)**.** These results corroborate the *in vitro* cellular data and confirm that DN200434 exhibits antiviral and anti-inflammatory cytokine efficacies against SARS-CoV-2 and IAV infection.

### (8) Synergistic antiviral efficacies of DN200434 in combination with remdesivir (SARS-CoV-2) or oseltamivir (IAV)

The administration of a combination of two antiviral drugs with distinct targets at lower concentrations can produce synergistic effects and reduce toxicity compared with a single antiviral drug at a high concentration. We evaluated the antiviral effects of DN200434 combined with two well-known antiviral agents, virus-targeted agents: remdesivir targeting SARS-CoV-2 RdRp and oseltamivir targeting IAV neuraminidase. In these experiments, hamsters received 20 mg/kg DN200434 and 2.5 mg/kg remdesivir daily, while mice received 10 mg/kg DN200434 and 2 mg/kg oseltamivir daily. Encouragingly, neither single nor combination therapy showed any signs of toxicity, including changes in body weight. The individual therapies with 40 mg kg⁻¹ d⁻¹ DN200434 or 2.5 mg kg⁻¹ d⁻¹ remdesivir resulted in 50% and 20% reductions of gross lung lesions, respectively. However, the combined administration of 20 mg kg⁻¹ d⁻¹ DN200434 and 2.5 mg kg⁻¹ d⁻¹ remdesivir resulted in remarkable reductions in gross lung lesions-relative to 100% lesions in the untreated control group-by 80%, 75%, and 78% in hamsters challenged with the KCDC03 strain (A lineage), KDCA51463 strain (alpha lineage), and KDCA55905 strain (beta lineage), respectively (**FIGS. 8I****,** **8J**)**.** Moreover, the combination therapy of 10 mg kg⁻¹ d⁻¹ DN200434 and 2 mg kg⁻¹ d⁻¹ oseltamivir in IAV-challenged mice resulted in a 90% survival rate (**FIGS. 8K****,** **8L**)**.** In parallel, the combination therapy effectively suppressed the weight loss associated with viral infections and provided superior alleviation of the clinical signs compared to individual therapies (**FIGS. 12A-12E**)**.** Taken together, our data suggest that the combination therapy, featuring the host-targeting DN200434 along with drugs that directly target the virus, results in synergic antiviral effects.

### 3. Discussion

The present invention reports that ERRy-mediated lipidomic reprogramming occurs through the transactivation of SREBP1c, which in turn provides FFAs to facilitate the replication of various RNA viruses, including SARS-CoV-2 and IAV. We found that RNA viral infections upregulate ERRy expression by activating the reactive oxygen species (ROS)-induced JNK/c-Jun signaling pathway. Virus-induced upregulation and translocation of ERRy to the nucleus directly transactivates SREBP1c gene expression, eventually driving an increase in FA biosynthesis and LD formation. Disrupting the action of ERRy by treatment with its inverse agonist DN200434 or by siRNA knockdown significantly blocked the replication of diverse RNA viruses by inhibiting SREBP1c-dependent FA biosynthesis and LD formation. The inhibitory effects are particularly through the inhibition of viral protein palmitoylation, reduced double-membrane vesicle (DMV) formation, and lower beta-oxidation-driven mitochondrial energy production, all of which are indispensable for viral replication. Finally, the administration of DN200434 significantly mitigated SARS-CoV-2-induced gross lung lesions in hamsters and protected mice from lethal IAV infection by blocking SREBP1c-mediated FA biosynthesis. Collectively, the present invention shows that targeting ERRy with DN200434, an ERRy inverse agonist, may protect against COVID-19, IAV infection, and RNA viral diseases that may emerge in the near future.

ERRy is known to be transactivated by ROS-induced JNK/c-Jun signaling pathway in the mouse liver. It is noted that in vitro and in vivo infections with many viruses are associated with elevated ROS levels. These lead us to predict that ROS induced by viral infection activates the JNK/c-Jun signaling pathway, ultimately transactivating ERRy. In the present invention, it has been confirmed that ROS activates the JNK/c-Jun signaling pathway in IAV- or SARS-CoV-2-infected cells, which ultimately transactivates ERRy. Also, ROS activation was suppressed in IAV- and SARS-CoV-2-infected cells by antioxidant treatment (NAC), resulting in a marked reduction in ERRy promoter activity. This indicates that the ERRy is indeed a ROS sensor in virus-infected cells. In particular, infection with either IAV or SARS-CoV-2 led to the phosphorylation of JNK and c-Jun, as well as to the activation of ERRy promoter activity, whereas the ERRy promoter with mutations in the AP1 regulatory element was not activated. Moreover, NAC treatment significantly inhibited c-Jun binding to the AP1 regulatory element site of the ERRy promoter. These suggest that ROS induced by viral infection activate the JNK/c-Jun signaling pathway, leading to transcriptional activation of ERRy.

SREBPs and their associated FA and cholesterol biosynthesis pathways play crucial roles in multiple steps of the viral life cycle. However, it remained unclear how SREBPs are activated during viral replication. In previous studies, we showed that ERRy activates the SREBP1c promoter, and here, we show that activation of SREBP1c by ERRy leads to a reconfiguration of FA biosynthesis that is essential for virus replication. We found that many RNA viruses activate ERRy, and the activated ERRγ translocates to the nucleus. Moreover, ERRy binds directly to the promoter of the SREBP1c gene in RNA virus-infected cells, thereby facilitating SREBP1c-dependent FA biosynthesis reprogramming in the virus-infected cells. Conversely, the inhibition of ERRy suppressed SREBP1c-dependent FA biosynthesis, resulting in the blockage of de novo FA synthesis. As a result, the formation of viral replication compartments (DMVs for SARS-CoV-2), viral morphogenesis (protein palmitoylation), and energy production were reduced, subsequently suppressing viral replication. In addition, under FFA-deficient conditions in which ERRy was inhibited by DN200434, supplementation with FA restored the replication of SARS-CoV-2 and IAV. Thus, we demonstrate that the upregulation of ERRy in response to RNA viral infections directly modulates SREBP1c-dependent FA biosynthesis, resulting in the reprogramming of host cell physiology that is essential for viral replication (**FIGS. 11A-****11G**), and ERRy is a potentially important target for therapeutic intervention against various RNA viruses, including SARS-CoV-2 and IAV.

Recent evidence reports that LDs also play a central role in an antiviral host response through localization with immune proteins, such as viperin,65,66, and enhancing innate immune pathways. In particular, the latter is required for an efficient interferon response following early viral infection or poly (I:C) treatment. In addition, we previously demonstrated that poly I:C treatment activates ERRy expression through the RIG-I/MDA5/JNK/AP1 signaling pathway, which in turn directly transactivates type I interferon genes. Although ERRy exhibits both proviral and antiviral effects during viral infection, our work clearly shows that the proviral effect is significantly greater than the antiviral effect. If the antiviral effect of ERRy is more potent than its proviral effect, inhibition of ERRy could enhance viral replication. However, when ERRy was inhibited by DN200434 or siRNA, the replication of various RNA viruses was suppressed. DN200434 administration alleviated lung lesions in SARS-CoV-2-challenged hamsters and protected mice challenged with a lethal IAV strain

. Moreover, ERRy KO mice exhibited resistance when challenged with lethal IAV, further supporting this conclusion.

In addition, inhibitors of lipogenic enzymes by using statins (inhibitors of 3-hydroxy-3-methylglutaryl-CoA reductase during cholesterol biosynthesis), TOFA (inhibitor of acetyl-CoA carboxylase during FA biosynthesis), and C75 (inhibitor of FAS during FA biosynthesis) are known to block viral replication. Taken together, these observations indicate that the upregulation of ERRy has a stronger proviral than antiviral effect, and that LDs and lipid components are used as an energy source to drive virus production, viral factories, viral replication compartments, and viral components.

The upsurge of proinflammatory cytokines, called a cytokine storm, plays a critical role in worsening pneumonia in patients with COVID-19 and influenza, eventually increasing the severity and mortality of infected patients. In this invention, in vitro and in vivo inhibition of ERRy with DN200434 significantly reduced eicosanoid and cytokine levels, pneumonia severity, and overall mortality compared to the non-treated control groups. Our data could be explained by two mechanisms by which inhibition of the eicosanoid and cytokine storm leads to remission of lung lesions and lower mortality. FFA-derived eicosanoid storm after endoplasmic reticulum stress response via viral infection itself or viral-induced cell debris could cause a cytokine storm.49 Therefore, treatment with DN200434 suppresses the SREBP1c-dependent FA biosynthesis, which produces the eicosanoids associated with cytokine storm. Infected non-immune cells and innate immune cells, such as macrophages and dendritic cells, recognize viral genomes and proteins as pathogen-associated molecular patterns (PAMPs) through pattern recognition receptors (PRRs), rapidly evoking antiviral responses by producing proinflammatory cytokines. In the present invention, treatment with DN200434 reduced FA levels in virus-infected cells by blocking ERRy-mediated SREBP1c-dependent FA biosynthesis. This adversely affects the formation of the viral replication compartment, viral morphogenesis, and energy production, all necessary for viral replication. Therefore, the second mechanism is that the decrease in the production of viral genomes and proteins as PAMPs by treatment with DN200434 may not be sufficient to induce a cytokine storm, as determined by PRRs in infected or immune cells. Overall, our findings illustrate that the inhibitory effects of DN200434 on cytokine storms warrant further consideration as priority candidates for clinical evaluation in COVID-19 and IAV trials with severe pneumonia cases.

Conventional antiviral drugs have traditionally been engineered to target viral proteins. However, various host proteins are utilized at each step of the viral life cycle. In particular, host proteins that are commonly exploited by diverse viruses can serve as attractive targets for the development of broad-spectrum, host-targeted antivirals. In the present invention, the genome structures of the used viruses are positive-stranded (SARS-CoV-2, BCoV, PEDV, PRRSV, and PSaV), negative single-stranded (IAV), or double-stranded (RVA). Their virion morphologies are capsid (RVA and PSaV), or envelope (SARS-CoV-2, BCoV, PEDV, PRRSV, and IAV), and their genome organizations are either linear (SARS-CoV-2, BCoV, PEDV, PRRSV, and PSaV) or segmented (IAV and RVA). Thus, the viruses used in the present invention may represent major RNA viruses that have circulated in the past, are currently circulating, and may circulate in the future. Interestingly, our invention has discovered that DN200434, an ERRγ inverse agonist, has broad-spectrum antiviral activity against different target viruses growing in multiple cell lines. Moreover, we have examined its effectiveness against the ongoing pandemic SARS-CoV-2 and its variants. Nuclear receptors (NRs) are highly susceptible to small-molecule modulation and are thus attractive targets for drug development. Notably, approximately 10-20% of FDA-approved medications currently target NRs. Our invention with DN200434, an inverse agonist of ERRy, not only shows significant promise in combating a wide spectrum of viruses but also offers broader defense against the emergence of drug-resistant viral strains and new pandemic strains, including notable examples such as IAV, SARS-CoV, MERS-CoV, and SARS-CoV-2.

In a previous report, we identified DN200434 as the most potent ERRy inverse agonist, with a functional IC₅₀ of 0.006 µM/L, which was 12-fold higher than that of GSK5182, an active metabolite of tamoxifen and an FDA-approved drug optimized for estrogen receptor (ER)- positive breast cancer. DN200434 was originally developed for the diagnosis and treatment of anaplastic thyroid cancer and showed high ERRy affinity (IC₅₀ = 0.04 µM/L). Moreover, DN200434 also exhibited in vitro and in vivo absorption, distribution, metabolism, excretion, and toxicity (ADMET) profiles. In the present in vivo experiments, animals treated with a maximum dose of DN200434 showed no clinical, gross, or histopathological side effects. In addition, DN200434 was detected in sufficient quantities in the lungs via the bloodstream. Therefore, we believe that DN200434 could have potential clinical applicability for the treatment of broad-spectrum RNA viral diseases, although it should first pass safety and clinical trials.

Combination therapy is an effective antiviral therapy that reduces toxicity and increases efficacy. Antiviral agents can be classified into two broad mechanisms of action: 1) those targeting viral proteins or nucleic acids and 2) those targeting essential host factor(s) for viral replication. In the present invention, combination therapy using DN200434 (10 mg kg-1 d-1) and oseltamivir (2 mg kg-1 d-1) for the treatment of IAV-infected mice; and combination therapy using DN200434 (20 mg kg-1 d-1) and remdesivir (2.5 mg kg-1 d-1) for the treatment of SARS-CoV-2-infected hamster showed that markedly reduced the replication of each virus and significantly decreased IAV-induced mortality and SARS-CoV-2-induced lung lesions. Future clinical studies using double or triple antiviral therapies with DN200434 as a backbone will be warranted for treating patients with severe acute viral infections such as COVID-19.

In summary, our invention reveals that early transactivation of ERRy via the ROS/JNK/c-Jun pathway in virus-infected host cells reprograms SREBP1c-dependent FA biosynthesis, an essential step for viral replication. Treatment with DN200434 specifically reverses the virus-induced lipogenic reprogramming, inhibiting the replication of various RNA viruses in vitro, protecting mice from lethal IAV infection, and mitigating lung lesions in hamsters challenged with SARS-CoV-2 and its variants. Moreover, a dual antiviral therapy of DN200434 with remdesivir for COVID-19 and oseltamivir for IAV infection augmented their effects in virus-infected mice and hamsters, respectively. Given its affinity, excellent ADMET properties, and broad-spectrum antiviral activity, DN200434 can be used as a novel broad-spectrum antiviral drug and may be used to treat viral diseases, such as COVID-19 and emerging pandemic viral infections, in the near future.

The above description of the present disclosure is provided for the purpose of illustration, and it would be understood by those skilled in the art that various changes and modifications may be made without changing the technical conception and essential features of the present disclosure. Thus, it is clear that the above-described examples are illustrative in all aspects and do not limit the present disclosure. For example, each component described as of a single type can be implemented in a distributed manner. Likewise, components described as distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. An antiviral pharmaceutical composition, comprising an estrogen-related receptor γ (ERRy) inhibitor as an active ingredient.

2. The pharmaceutical composition of claim 1,
wherein the ERRy inhibitor inhibits the activity of the ERRy protein or suppresses the expression of the ERRy gene.

3. The pharmaceutical composition of claim 1,
wherein the ERRy inhibitor exerts antiviral effects by inhibiting ERRy activity in virus-infected cells.

4. The pharmaceutical composition of claim 1,
wherein the ERRy inhibitor includes an siRNA specific to ERRy.

5. The pharmaceutical composition of claim 1,
wherein the ERRy inhibitor includes an inverse agonist of ERRy.

6. The pharmaceutical composition of claim 5,
wherein the ERRγ inverse agonist includes a compound represented by the following Chemical Formula 1: 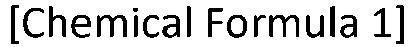 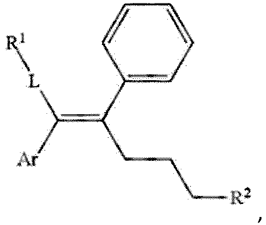
In Chemical Formula 1,
L is (C₆-C₂₀)arylene, (C₃-C₂₀)heteroarylene, or (C₃-C₂₀) fused heterocycle;
R¹is (C₃-C₂₀)heterocycloalkyl, (C₃-C₂₀)heteroaryl, -O-(CH₂)ₘ-R¹¹, -(CH₂)ₘ-R¹², -NH-(CH₂)ₘ-R¹³, -NHCO-(CH₂)ₙ-R¹⁴, or -SiR¹⁶R¹⁷-(CH₂)ₘ-R¹⁵;
R¹¹ to R¹⁵ are each, independently, (C₃-C₂₀)heterocycloalkyl;
R¹⁶ and R¹⁷ are each, independently, (C₁-C₂₀)alkyl;
m is an integer of 1 to 3; and
n is an integer of 0 or 1;
Ar is (C₆-C₂₀)aryl or (C₃-C₂₀)heteroaryl,
in which the aryl or heteroaryl of Ar is unsubstituted or substituted with one or more substituents selected from hydroxy, halogen, (C₁-C₂₀)alkyl, halo(C₁-C₂₀)alkyl, (C₁-C₂₀)alkoxy, nitro, cyano, -NR²¹R²², (C₁-C₂₀)alkylcarbonyloxy, (C₁-C₂₀)alkylcarbonylamino, guanidino, -SO₂-R²³, and -OSO₂-R²⁴,
R²¹ and R²² are each, independently, hydrogen, (C₁-C₂₀)alkylsulfonyl, or (C₃-C₂₀)cycloalkylsulfonyl;
R²³ and R²⁴ are each, independently, (C₁-C₂₀)alkyl, halo(C₁-C₂₀)alkyl, or (C₃-C₂₀)cycloalkyl;
R² is hydroxy, halogen, (C₁-C₂₀)alkylcarbonyloxy, or (C₁-C₂₀)alkylsulfonyloxy;
the heterocycloalkyl or heteroaryl of R¹ and the heterocycloalkyl of R¹¹ to R¹⁵ are unsubstituted or substituted with one or more substituents selected from (C₁-C₂₀)alkyl, (C₃-C₂₀)cycloalkyl, (C₂-C₂₀)alkenyl, amidino, (C₁-C₂₀)alkoxycarbonyl, hydroxy, hydroxy(C₁-C₂₀)alkyl, and di(C₁-C₂₀)alkylamino(C₁-C₂₀)alkyl, and
the heterocycloalkyl and heteroaryl contain one or more heteroatoms selected from N, O and S, and the heterocycloalkyl is a saturated or unsaturated mono-, bi-, or spirocycle having a carbon atom or nitrogen atom in a ring as a binding site.

7. The pharmaceutical composition of claim 5,
wherein the ERRγ inverse agonist includes a compound represented by the following Chemical Formula 2: 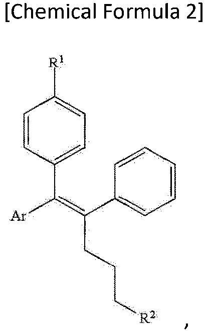
In Chemical Formula 2,
R¹ is (C₃-C₂₀)heterocycloalkyl, (C₃-C₂₀)heteroaryl, -O-(CH₂)ₘ-R¹¹, -(CH₂)ₘ-R¹², -NH-(CH₂)ₘ-R¹³, -NHCO-(CH₂)ₙ-R¹⁴, or -SiR¹⁶R¹⁷-(CH₂)ₘ-R¹⁵;
R¹¹ to R¹⁵ are each, independently, (C₃-C₂₀)heterocycloalkyl;
R¹⁶ and R¹⁷ are each, independently, (C₁-C₂₀)alkyl;
m is an integer of 1 to 3; and
n is an integer of 0 or 1;
Ar is (C₆-C₂₀)aryl or (C₃-C₂₀)heteroaryl,
in which the aryl or heteroaryl of Ar is unsubstituted or substituted with one or more substituents selected from hydroxy, halogen, (C₁-C₂₀)alkyl, halo(C₁-C₂₀)alkyl, (C₁-C₂₀)alkoxy, nitro, cyano, -NR²¹R²², (C₁-C₂₀)alkylcarbonyloxy, (C₁-C₂₀)alkylcarbonylamino, guanidino, -SO₂-R²³, and -OSO₂-R²⁴,
R²¹ and R²² are each, independently, hydrogen, (C₁-C₂₀)alkylsulfonyl, or (C₃-C₂₀)cycloalkylsulfonyl;
R²³ and R²⁴ are each, independently, (C₁-C₂₀)alkyl, halo(C₁-C₂₀)alkyl, or (C₃-C₂₀)cycloalkyl;
R² is hydroxy, halogen, (C₁-C₂₀)alkylcarbonyloxy, or (C₁-C₂₀)alkylsulfonyloxy;
the heterocycloalkyl or heteroaryl of R¹ and the heterocycloalkyl of R¹¹ to R¹⁵ are unsubstituted or substituted with one or more substituents selected from (C₁-C₂₀)alkyl, (C₃-C₂₀)cycloalkyl, (C₂-C₂₀)alkenyl, amidino, (C₁-C₂₀)alkoxycarbonyl, hydroxy, hydroxy(C₁-C₂₀)alkyl, and di(C₁-C₂₀)alkylamino(C₁-C₂₀)alkyl, and
the heterocycloalkyl and heteroaryl contain one or more heteroatoms selected from N, O and S, and the heterocycloalkyl is a saturated or unsaturated mono-, bi-, or spirocycle having a carbon atom or nitrogen atom in a ring as a binding site.

8. The pharmaceutical composition of claim 5,
wherein the ERRγ inverse agonist includes DN200434 and/or GSK5182.

9. The pharmaceutical composition of claim 1,
wherein the pharmaceutical composition is for the prevention or treatment of RNA virus infections.

10. The pharmaceutical composition of claim 9,
wherein the RNA virus includes at least one selected from SARS-CoV-1, SARS-CoV-2, influenza A virus (IAV), influenza B virus, bovine coronavirus (BCoV), porcine epidemic diarrhea coronavirus (PEDV), bovine species A rotavirus (RVA), porcine reproductive and respiratory syndrome virus (PRRSV), porcine sapovirus (PSaV), norovirus (NoV), feline infectious peritonitis virus (FIPV), MERS-related coronavirus (MERS-CoV), Zika virus (ZIKV), dengue virus, human torovirus (HuTV), hepatitis A virus (HAV), and hepatitis C virus (HCV).

11. The pharmaceutical composition of claim 1,
further comprising an antiviral agent.

12. The pharmaceutical composition of claim 11,
wherein the antiviral agent includes one or more selected from oseltamivir and remdesivir.

13. The pharmaceutical composition of claim 12,
wherein the antiviral agent is oseltamivir,
the volume ratio of the ERRy inverse agonist to oseltamivir is 2:2 to 8:2.

14. The pharmaceutical composition of claim 12,
wherein the antiviral agent is remdesivir,
the volume ratio of the ERRy inverse agonist to remdesivir is 8:1 to 20:1.
